(19) Europäisches Patentamt
European Patent Office
Office européen des brevets

(11) EP 0 404 097 B1

(12) **EUROPÄISCHE PATENTSCHRIFT**

(45) Veröffentlichungstag und Bekanntmachung des Hinweises auf die Patenterteilung:
**04.09.1996 Patentblatt 1996/36**

(51) Int. Cl.$^6$: **C07K 16/18**, C12N 15/13, C12P 21/00

(21) Anmeldenummer: **90111640.0**

(22) Anmeldetag: **20.06.1990**

(54) **Bispezifische und oligospezifische, mono- und oligovalente Rezeptoren, ihre Herstellung und Verwendung**

Bispecific and oligospecific, mono- and oligovalent receptors, production and applications thereof

Récepteurs mono- et oligovalents, bispécifiques et oligospécifiques, ainsi que leur production et application

(84) Benannte Vertragsstaaten:
**AT BE CH DE DK ES FR GB GR IT LI LU NL SE**

(30) Priorität: **22.06.1989 DE 3920358**

(43) Veröffentlichungstag der Anmeldung:
**27.12.1990 Patentblatt 1990/52**

(73) Patentinhaber: **BEHRINGWERKE Aktiengesellschaft**
**35001 Marburg (DE)**

(72) Erfinder:
- **Bosslet, Klaus, Dr.**
  **D-3550 Marburg (DE)**
- **Hermentin, Peter, Dr.**
  **D-3550 Marburg (DE)**
- **Seemann, Gerhard, Dr.**
  **D-3550 Marburg (DE)**
- **Kuhlmann, Ludwig, Dr.**
  **D-6093 Flörsheim am Main (DE)**
- **Steinsträsser, Axel, Dr. Dr.**
  **D-6237 Liederbach (DE)**

(56) Entgegenhaltungen:
**EP-A- 0 141 079**
**EP-A- 0 369 566**
**WO-A-86/01407**
**US-A- 4 652 440**

- **JOURNAL OF IMMUNOLOGY, Band 141, Nr. 11, 11. Dezember 1988; C.B. BEIDLER et al. , Seiten 4053-4060**
- **TRENDS IN BIOTECHNOLOGY, Band 6, Nr. 2, Februar 1988, Cambridge (GB); G. WILLIAMS, Seiten 36-39, 42**
- **BIOTECHNIQUES, Band 4, Nr. 3, Mai/Juni 1986; V.T. OI et al., Seiten 214-221**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.4/3.4

## Beschreibung

Die Erfindung betrifft bispezifische und oligospezifische, mono- und oligovalente Rezeptoren, die gentechnisch durch Fusion von für F(ab) Fragmente von Antikörper zweier oder mehrerer verschiedener Spezifitäten kodierende DNA mittels geeigneter Linker hergestellt werden dadurch gekennzeichnet, daß eine Spezifität über fos-jun Interaktion gegen ein Komplexon gerichtet ist. Vorzugsweise ist eine Spezifität dabei entweder gegen ein auf der Zellmembran oder im Interstitium befindliches Epitop eines Tumor-assoziierten Antigens (TAA) oder gegen ein Epitop im Tumorendothel (TE) gerichtet, während die weiteren Spezifitäten hochmolekulare oder niedermolekulare Liganden betreffen und z.B. mit den Komplexonen Äthylendiamintetraacetat bzw. Diäthylentriaminpentaacetat in Y90 komplexierter Form (EDTA-Y90 bzw. DTPA-Y90) reagieren. Die Bindung mit den Komplexonen erfolgt am Komplexon-Rezeptor-Arm über fos-jun Interaktion (oder auch Avidin-Biotin Interaktion). Weitere bevorzugte Spezifitäten haben katalytische Eigenschaften.

Bispezifische Antikörper wurden bisher nach folgenden Methoden hergestellt

- chemische Kopplung von Antikörpern verschiedener Spezifität über heterobifunktionelle Linker (H. Paulus, Behring Inst. Mitt. 78, (1985), 118-132)
- Fusion von bereits vorhandenen Hybriden, die verschiedene monoklonale Antikörper (MAK) ausscheiden, und Isolation des bispezifisch-monovalenten Anteiles (U.S. Staerz und M. J. Bevan, Proc. Natl. Acad. Sci. USA 83, (1986) 1453-1457
- Transfektion der leichten und schweren Kettengene zweier verschiedener MAK (4 Gene) in murine Myelomzellen oder andere eukaryotische Expressionssysteme und Isolation des bispezifisch-monovalenten Anteiles (U. Zimmermann, Rev. Physio. Biochem. Pharmacol. 105 (1986), 176-260; J. van Dijk et al., Int. J. Cancer 43, (1989), 944-349).

Solche bispezifische Antikörper werden zur Therapie und Diagnostik von malignen Tumoren eingesetzt. Das Prinzip des Verfahrens besteht darin, daß im ersten Schritt durch Injektion des bispezifischen Makromoleküls über längere Zeiträume und mit hohen Dosen eine Absättigung der Epitope, die von einer der beiden Spezifitäten auf den Zielzellen erkannt werden, erreicht wird. Im zweiten Schritt, der aus einer mehrtägigen Unterbrechung der Behandlung besteht, findet die Autoelimination des unspezifisch adsorbierten bispezifischen Antikörpers aus den nicht Zielgeweben statt.

Diese Autoelimination kann durch Injektion eines mit Zuckerresten, vorzugsweise Galactose gekoppelten anti-idiotypischen Antikörpers, der gegen den Anti-Tumor-Arm des bispezifischen Rezeptors gerichtet ist, beschleunigt werden.

Der dritte Schritt des Verfahrens besteht in der i.v. Injektion eines radiomarkierten, hydrophilen, sich nicht in Zellen anreichernden niedermolekularen Liganden mit kurzer Verweilzeit im Organismus, der hohe Komplexkonstanten für beta- und gamma-Strahler wie $Y^{90}$, $Re^{186}$, $Re^{188}$, $Re^{189}$, $^{99m}Tc$ oder $^{111}In$ hat und an den die zweite Spezifität des bispezifischen Antikörpers mit hoher Affinität bindet. Durch diesen Schritt wird eine Anreicherung des radioaktiven Liganden, verbunden mit längerem Verbleib am Zielgewebe erreicht, was die selektive Zerstörung des Zielgewebes zur Folge hat bzw. eine Diagnostik beispielsweise von Metastasen ermöglicht.

Die Erfindung stellt nun bispezifische bzw. oligospezifische Rezeptoren bereit, die je nach Erfordernis mono- oder oligovalente Bindungen zu den jeweiligen Epitopen besitzen und gentechnisch mittels geeigneter "Linker" erzeugt werden. Dabei werden die Genfragmente, die für die $V_H$ und $C_H1$ Abschnitte der Antikörper a und b kodieren, mittels geeigneter synthetischer Oligonukleotide wie in Tab. 1 beispielhaft dargestellt, dergestalt verknüpft, daß der N-Terminus der $V_H$ Dömane des MAK b über einen Polypeptidspacer mit dem C-Terminus der $C_H1$ Domäne des MAK a kovalent verbunden ist (Fig. 1). Das Genkonstrukt aus $V_HaC_H1a$-Polypeptidspacer-$V_HbC_H1b$ wird zusammen mit den Genen für die leichten Ketten der Antikörper a und b in eukaryontische Zellen transfiziert (z.B. Mausmyelomzellen). Die $C_H1a$, $C_H1b$, $C_ka$ und $C_kb$ Domänen sind so modifiziert, daß an den Kontaktflächen der konstanten Domänen entgegengesetzte Ladungen aufeinandertreffen ($C_H1a(+)C_ka(-)$; $C_H1b(-)C_kb(+)$) (+ = positiv, - = negativ) bzw. sich jeweils hydrophobe oder jeweils hydrophile Kontaktflächen gegenüberstehen. Dadurch werden von den Transfektomas bevorzugt Hybridmoleküle ausgeprägt, bei denen die korrekten Paarungen aus schweren und leichten Ketten vorliegen (Fig. 2).

Antikörper a steht hier beispielhaft für einen Antitumor-Antikörper, Antikörper b für einen Antikörper gegen einen niedermolekularen Liganden, vorzugsweise die Komplexone DTPA-Y90 oder EDTA-Y90.

Bi- oder oligospezifischer Rezeptor bedeutet demnach eine gentechnische Konstruktion aus $V_H$ und $C_H1$ Domänen von Antikörpern verschiedener Spezifität über geeignete Linker, so daß die erforderliche Beweglichkeit zur Assoziation mit den korrespondierenden leichten Ketten gegeben und die Antigenbindung nicht behindert ist.

Valenzen oder Bindungsstellen bezeichnen die Antigenbindungsstellen. Ein bispezifischer monovalenter Rezeptor hat bei zwei Spezifitäten, also je eine Antigenbindungsstelle. Ein bispezifischer trivalenter Rezeptor besitzt folglich eine Antigenbindungsstelle für die eine Spezifität und zwei Antigenbindungsstellen für die andere.

Bispezifische Rezeptoren, die bivalent für das Tumorantigen (MAK a) und monovalent für EDTA-Y90 (MAK b) sind, werden hergestellt, indem man das oben beschriebene schwere Ketten-Genkonstrukt mittels des oben erwähnten Oligonukleotidlinkers mit dem Genabschnitt verknüpft, der für die $V_H$ und $C_H1$ Domäne des MAK a kodiert (Fig. 3), so daß

das C-terminale Ende der $C_H1$ Domäne des MAK b mit dem N-terminalen Ende der $V_H$ Domäne des MAK a durch einen Polypeptidspacer verbunden ist. Diese Genkonstrukte werden zusammen mit den Genen für die zu den MAK a und b gehörenden leichten Ketten in eukaryontische Zellen transfiziert (z.B. Myelomzellen). Die $C_H1$ und $C_k$ Domänen sind, wie oben beschrieben, mit komplementären Ladungen bzw. jeweils hydrophoben oder hydrophilen Kontaktflächen versehen. Von den Transfektomas werden bevorzugt Fusionsmoleküle ausgeprägt, die aus zwei F(ab) Fragmenten des MAK a und einem F(ab) Fragment des MAK b bestehen Fig. 4). Die Beweglichkeit der Peptidlinker ermöglicht die Ausrichtung der beiden F(ab)-Arme des MAK a zur Tumorzelle bei gleichzeitiger Ausrichtung des F(ab)-Armes des MAK b zum interzellulären Raum. Entsprechend können weitere Bindungsstellen identischer oder anderer Spezifität zugefügt werden. Die Reihenfolge der Spezifitäten in den Konstrukten ist dabei frei kombinierbar.

Die Erfindung betrifft folglich bispezifische oder oligospezifische, mono- oder oligovalente Rezeptoren, die sowohl Spezifität für ein auf der Zellmembran oder im Interstitium befindliches Epitop haben, zum Beispiel TAA oder TE, als auch Spezifitat für einen nieder- oder hochmolekularen Liganden besitzen, der sich ausschließlich im extrazellulären Raum verteilt. Eine Spezifität wird dabei vorzugsweise von den tumorspezifischen Antikörpern gebildet, wie in der deutschen Patentanmeldung P 39 09 799.4 vorgeschlagen, während die andere Spezifität vorzugsweise gegen DTPA-Y90 oder EDTA-Y90 gerichtet ist. und die Bindung mit Komplexonen am Komplexon-Rezeptor-Arm über fos-jun Interaktion (siehe Beispiel 5) erfolgt. Eine bevorzugte Variante der Erfindung besteht im Einbau von katalytisch wirksamen Spezifitäten. Die Reihenfolge der Spezifitäten bzw. Bindungsvalenzen ist dabei frei wählbar, wie beispielhaft in Fig. 4 für drei Valenzen bei zwei Spezifitäten gezeigt.

Besonders bevorzugt bei den erfindungsgemäßen Konstrukten sind solche, die ein V-Gen der Tabellen 2, 3, 4 und/oder 5 enthalten. Antikörper mit diesen Sequenzen und ihre Eigenschaften sind in der deutschen Patentanmeldung P 39 09 799.4 beschrieben. Die "Complementarity Determining Regions" (CDR's) sind dabei nach Kabat und Wu (Sequences of Proteins of Immunological Interest, US Dept. of Health and Human Services, US Government Printing Office (1987)) identifizierbar. Bevorzugt sind ebenfalls Konstrukte, die Spezifitäten gegen die durch vorstehend geschilderte monoklonale Antikörper definierte Epitope enthalten.

Zusätzlich betrifft die Erfindung gentechnische Verfahren zur Herstellung oben beschriebener Konstrukte sowie eine Verwendung o.g. Konstrukte zur Herstellung von Arzneimitteln zur Bekämpfung und Diagnose von Zielzellen. Dabei findet in einem 1. Schritt nach Injektion der Konstrukte eine Absättigung der betroffenen Epitope auf Zielzellen statt, in einem nachfolgenden Intervall werden unspezifisch adsorbierte oder nicht gebundene Konstrukte eliminiert. Der daran anschließende Schritt besteht in der Injektion und nachfolgender spezifischer Bindung eines sich nicht in den Zielzellen anreichernden nieder- oder hochmolekularen Liganden, der per se zytotoxisch ist oder gegebenenfalls in einem weiteren Schritt durch extrakorporale Einwirkungen zur Zytoxizität "aktiviert" wird. Verfahren dieser Art sind beispielsweise enzymatische Aktivierung, Aktivierung durch Mikrowellenbestrahlung eines "Pro-Drugs" oder Aktivierung durch Laserlicht.

Die Erfindung ist ferner in den Beispielen und den Patentansprüchen enthalten.

Beispiel 1: Herstellung eines anti-DTPA-Y90 bzw. EDTA-Y90-MAK

Als Hapten wurde Isothiocyanato-benzyl-DTPA (Formel 2) auf humanes Serumalbumin (HSA als Carrier) mit einem Derivatisierungsgrad von 19 Benzyl-DTPA Molekülen pro HSA-Molekül entsprechend der in (N.W. Brechbiel et al., Inorganic Chemistry 25, (1986) 2772-2781) beschriebenen Methodik kovalent gekoppelt. 20 µg dieses Hapten-Carrier-Komplexes, in welchen kaltes Y komplexiert wurde, wurden s.c. an Tag 0 mit Freundschem Adjuvans, an Tag 7 und 14 mit inkomplettem Freundschem Adjuvans und an Tag 21 mit PBS in Balb/c Mäuse injiziert. An Tag 24 wurden die Milzen der Mäuse mit den höchsten anti DTPA Antikörpertitern mit der SP2/0-Ag14 Myelomzellinie (Shulman et al., Nature 276, (1978) 269) fusioniert. Entstehende Hybridome wurden in einem DTPA-spezifischen ELISA auf Produktion von hochaffinen MAK gegen DTPA und EDTA überprüft. Der ELISA bestand aus einer festen Phase, die mit einer HSA-Benzyl-DTPA-Y enthaltenden Lösung beladen wurde. Der zu testende, den MAk enthaltende Überstand wurde mit freiem Komplexon bzw. dessen Metallionkomplexen vorinkubiert und seine Bindung an die spezifische feste Phase gemessen. Hierzu wurde ein Enzymamplifikationssystem benutzt, welches an einen anti Maus-Immunglobulinantikörper gekoppelt ist. Die Details dieser Methodik sind in Anlage 1a und 1b beschrieben.

Mittels dieses Testsystems wurden MAk erhalten, welche die in Anlage 1e beschriebenen Eigenschaften besitzen.

Diese MAk binden im Gegensatz zu vielen anderen anti DTPA/EDTA MAk nicht an menschliche Normalgewebe, wie mittels der APAAP-Technik (Cordell et al., J. Histochem. Cytochem. 32: 219, 1984) auf kryopräservierten Geweben ermittelt wurde. Ein in vivo Einsatz dieser MAk im Bereich der Diagnostik und Therapie ist somit möglich.

Als Kompetitoren wurden die Komplexone DTPA, EDTA in nicht komplexierter sowie in komplexierter Form (Anlage 1c) eingesetzt. Zusätzlich wurden als Inhibitoren die strukturverwandten Verbindungen Transaconitsäure und 1,2 Diaminoethan verwendet (siehe Anlage 1e). Besonders geeignet für eine in vivo Anwendung ist der MAk BW 2050/174 der im Gegensatz zu allen anderen MAk eine präferentielle Bindung an EDTA-Y zeigt (siehe Anlage 1e, niedriger Kompetitor Überschuß für EDTA-Y (100fach) höherer Überschuß für andere EDTA-Komplexone. Deshalb wurde das Hybrid 2050/174 stabilisiert und für die Entwicklung des EDTA-Y-Armes im bispezifischen Rezeptor verwendet.

Beispiel 2: Herstellung und Expression eines $V_H$1a $C_H$1-Linker-$V_H$1B $C_H$1 Genkonstruktes

Die hier verwendeten Techniken wurden, wenn nicht anders angezeigt, aus Molecular Cloning, a Laboratory Manual; Sambrook, Fritsch, Maniatis; Cold Spring Habor Laboratory, 1982 (S. 11 - 44, 51 - 127, 133 - 134, 141, 146, 150 - 167, 170, 188 - 193, 197 - 199, 248 - 255, 270 - 294,310 - 328, 364 - 401, 437 - 506) und aus Molecular Cloning, A Laboratory Manual, Second Edition; Sambrook, Fritsch, Maniatis; Cold Spring Harbor Laboratory Press, 1989, (S. 16.2 - 16.22, 16.30 - 16.40, 16.54 - 16.55) entnommen.

Ein humanes $IgG_3$ C-Gen wurde aus einer humanen Genbank in EMBL3 Phagen isoliert (A.M. Frischauf et al., J. Mol. Biol. 170, 827-842 (1983) und G.H.A. Seemann et al., The EMBO Journal 5 (1986), 547-552).

Aus diesem $IgG_3$ C-Gen wurden wie in der deutschen Patentanmeldung P 38 25 615.0 beschrieben, Konstruktionen hergestellt, die zum einen nur noch das $C_H$1 Exon und ein Hinge Exon (Fig. 5) und zum anderen das $C_H$1 Exon und den 3′NT Bereich eines HLA B27 Gens enthalten (Fig. 6, Fragment M im Plasmid M).

Die $V_H$a und $V_H$b Gene wurden aus mRNA der Hybridklone a und b amplifiziert, wie von Orlandi et al. (Proc. Natl. Acad. Sci, USA 86, (1989), 3833-3837) beschrieben und in einen M13 Vektor kloniert ($V_H$a PCR bzw. $V_H$b PCR) (Fig. 7). Das $V_H$a Gen wurde als HindIII BamH1 Fragment in den eukaryontischen Expressionsvektor p$EV_H$ kloniert (Simon et al., Nucl. Acids. Res. 16, (1988), 354) (Fig. 8). Es entsteht das Plasmid $PEV_H$ a C.

Der humane IgG C Gen Subklon mit dem $C_H$1 und dem einen Hinge Exon (Fig. 5) enthält zwischen $C_H$1 Exon und Hinge Exon eine Pst1 Schnittstelle. Die $V_H$ Gene enthalten am 5′ Ende eine Pst1 Schnittstelle. Das Linkeroligonukleotid wird so entworfen, daß es am 5′ Ende mit dem Bereich der Pst1 Schnittstelle auf dem $C_H$1 + 1H Subfragment des IgG C Gens und am 3′ Ende mit der Pst1 Schnittstelle des VHb Gens überlappt. Das Linkeroligonukleotid wird mittels seiner Pst1 Schnittstellen in die Pst1 Schnittstelle eines PUC 18 Plasmids kloniert (Fig. 9). Es entsteht der Plasmidklon L.

Das Plasmid mit dem $IgG_3$ C Gen Subfragment mit $C_H$1 Exon und einem Hinge Exon wird mit Pst1 und BamH1 gespalten und mit dem als Pst1 BamH1 Fragment aus $V_H$b PCR ausgeschnittenem $V_H$b Genfragment ligiert (Fig. 10). Es entsteht das Plasmid X.

Das Plasmid X wird mit Pst1 gespalten und mit dem ebenfalls mit PstI aus dem Plasmid L ausgeschnittenen Linkerfragment ligiert (Fig. 11). Mit Hilfe der Nukleinsäuresequenzanalyse wird der Klon Z identifiziert, bei dem der Linker in der richtigen Orientierung zwischen $C_H$1 und $V_H$b kloniert ist, ohne den Intron/Exonübergang zwischen Intron 3 und Linkerexon und ohne das Leseraster am Übergang zwischen Linker und $V_H$b Gen zu stören.

Das Plasmid pEVa C wird mit BamH1 gespalten und mit dem mit BamH1 aus dem Plasmid M ausgeschnittenen Fragment M ligiert. Durch Restriktionsanalyse wird der Klon Y identifiziert, der das Fragment M in der richtigen Orientierung enthält (Fig. 12).

Das Plasmid Y wird partiell mit BamH1 gespalten und mit dem durch HindIII und BamH1 Spaltung aus dem Plasmid X ausgeschnittenen Fragment ($C_H$1-Linker-$V_H$b) nach Auffüllen aller Enden ligiert. Durch Nukleotidsequenzanalyse und Restriktionskartierung wird der Plasmidklon PEVT identifiziert, der das Fusionsgen $V_H$a $C_H$1-Linker-$V_H$b $C_H$1 mit der richtigen Orientierung aller Exons enthält (Fig. 13).

Das Plasmid PEVT wird zusammen mit Plasmiden, die die Gene für die leichten Ketten der Antikörper a bzw. b tragen, in geeignete eukaryontische Expressionszellen transfiziert, um das Antikörper a F(ab) Antikörper b F(ab) Fusionsprotein auszuprägen.

Beispiel 3: Transfektion der leichten und schweren Ketten-Gene zweier verschiedener MAk (4 Gene)

Die Isolierung von Immunglobulingenen ist in der deutschen Patentanmeldung P 39 09 799.4 beschrieben.

Die in Vektoren klonierten Gene wurden mittels Elektroporation nach Linearisierung der Vektoren in X63Ag8.653 Myelomzellen transfiziert (H. Stopper et al., Biochem. Biophys. Acta 900 (1987), 38-44). Die in Selektivmedien ausgewachsenen Transfektome wurden auf Produktion von bispezifischem-monovalentem MAK in einem spezifischen RIA überprüft. Dieser RIA bestand aus Festphasen-adsorbiertem TAA, auf welchen nach Blockade der unspezifischen "sites" durch Casein die zu testenden Transfektomüberstände gegeben wurden. Nach Zugabe von mit $Y^{90}$ oder $^{99m}$Tc komplexiertem DTPA oder EDTA und Wegwaschen des Überschusses konnten diejenigen Transfektome, die bispezifischen-monovalenten anti TAA x anti EDTA MAK ausschieden, an einem erhöhten radioaktiven Signal auf der Festphase detektiert werden.

Transfektom 9 wurde stabilisiert durch "limited dilution" Klonierung und in Zellkultur aufgebaut. Zellkulturüberstände wurden 10fach ankonzentriert, der MAK-Anteil wurde über Protein A chromatographiert (P.L. Ey et al., Immunochemistry 15, (1978), 429) gereinigt und der den bispezifisch-monovalenten MAK enthaltende Anteil mittels Anionenaustauscher-Chromatographie gereinigt. (J. Van Dijk et al., Int. J. Cancer 43, (1989), 344-349).

Beispiel 4: Biologische Wirksamkeit

Gereinigtes, den bispezifischen-monovalenten MAK (BW 431/26 x BW 2050/174) enthaltendes Protein wurde in 500 µg Dosen an den Tagen 0, 3, 5, 8, 10 und 12 i.v. in human Tumorxenografts (CoCa 4) tragende Nacktmäuse injiziert. An Tag 27-30 erhielten die Tiere je 50 µCi EDTA-Y90 i.v. injiziert. Eine 2. Gruppe von Tieren erhielt an den gleichen Tagen anstatt des bispezifischen MAK 500 µg des MAK BW 431/26 und, wie oben beschrieben, die EDTA-Y90-Injektionen.

Eine dritte mit Tumor bestückte Gruppe erhielt anstatt des MAK und des EDTA-Y90 Injektionen von PBS (Tumorwachstumskontrolle).
Das Tumorwachstum wurde über 6 Wochen verfolgt. Die Injektion von EDTA-Y90 führte in der Gruppe, die den bispezifischen-monovalenten MAK erhielt, zu einer signifikanten Tumorwachstumsinhibition, wohingegen die mit MAK BW 431/26 injizierten und mit EDTA-Y90 behandelten Tiere keine Tumorwachstumsinhibition zeigten, im Vergleich zu den Tieren, die nur PBS erhielten.

Diese Daten weisen auf die selektive tumortherapeutische Wirksamkeit des bispezifischen-monovalenten MAK in Kombination mit EDTA-Y90 als toxischem Prinzip hin.

Noch günstigere tumortherapeutische Effekte werden durch die oligovalenten/bispezifischen oder oligospezifischen Rezeptoren erhalten, da sie aufgrund der bivalenten Bindung an TAA länger am Tumor verweilen und somit der Ligand ebenfalls länger und in höherer Konzentrationen am Tumor zurückgehalten wird.

Beispiel 5: Optimierung der biologischen Wirksamkeit bi-oder oligospezifischer Makromoleküle durch Aviditätserhöhung des anti-Komplexonarmes.

Ein entscheidender Faktor, der die effiziente Anheftung des hydrophilen, sich extrazellulär verteilenden Komplexons am anti-Komplexonarm des oligospezifischen Makromoleküls beeinflußt, ist die Avidität dieses Armes zum Komplexon. Die Aviditäten von monoklonalen Antikörpern zu ihren entsprechenden Epitopen liegen im Bereich von $10^5$ - $10^{11}$ l/mol. Da diese Bindungsstärken vielleicht nicht ausreichen, um die für eine effiziente Radioimmuntherapie notwendige Komplexonmasse am Tumor zu lokalisieren, wurde im folgenden Beispiel die extrem starke Interaktion des fos-Leucin-Reißverschlußpeptids (fos-Peptid) mit dem jun-Leucin-Reißverschlußpeptid (jun-Peptid) genutzt (Erin K. O'Shera et al., Science, 245, 1989), um das Komplexon möglichst fest am anti Komplexonarm zu fixieren. Um diese starke fos-jun Interaktion nutzen zu können, muß vorzugsweise das fos-Peptid kovalent mit dem Komplexon (DTPA) verknüpft werden. Hierzu kann im ersten Schritt isothiocyanatobenzyl DTPA mit Hydrazin (oder einem Diaminoalkan) umgesetzt werden. Das so entstandene DTPA-Benzyl-thiocarbazid kann in einem 2. Schritt mit N-(gamma-maleimidobutyryloxy)succinimid oder einem Analogon zu DTPA-Benzyl-(gamma-maleimidobutyryl) thiocarbazid umgesetzt werden. In einem 3. Schritt wird dann diese Verbindung mit dem um Glycin-Glycin-Cystein verlängerten fos-Peptid (Bild 1) über die freie SH-Gruppe des aminoterminalen Cysteins verknupft. Das so entstandene fos-Peptid-DTPA-Konjugat wird in einem 4. Schritt mit Yttriumchlorid komplexiert. Der so entstandene fos-Peptid-DTPA-Y-Konjugatkomplex kann dann zur in vivo Anlagerung an den jun-peptid-Arm des bi- oder oligo-spezifischen Makromoleküls benutzt werden. Nachfolgend ist die Synthese des oben vorskizzierten Beispiels im Detail beschrieben:

A) Herstellung des fos-EDTA-Y-Konjugat-Komplexes

Schritt 1:

Synthese von EDTA-Benzyl-thiocarbazid

Isothiccyanatobenzyl-EDTA (SCN-Bn-ETPA) (30 mg, 54 µmol) wurde 1 h lang in 10 % (v/v) wäßrigem Hydrazin gerührt. Dann wurde das Losungsmittel im Hochvakuum entfernt, der Rückstand über Phosphorpentoxid im Hochvakuum getrocknet und schließlich lyophilisiert. Das Produkt wurde mit DOWEX WX 2 (H+-Form) neutralisiert und erneut lyophilisiert (Ausbeute 28 mg).

Schritt 2:

Synthese von EDTA-Benzyl-(gamma-maleimidobutyryl)thiocarbazid

Das in Schritt 1 hergestellte ETPA-Benzyl-thiocarbazid (20 mg; 34 µmol) und N-(gamma-Maleimidobutyryloxy)succinimid (8 mg, 29 µmol = 0.9 equiv.) wurden 1 h lang in wasserfreiem Dimethylformamid gerührt. Dann wurde zur Trockne eingeengt und der Rückstand im Hochvakuum getrocknet.

Schritt 3:

Kopplung des EDTA-Benzyl-(gamma-maleimidobutyryl)thiocarbazid an aminoterminales Cystein im fos-Peptid

Eine Lösung des fos-peptids (4.8 mg, 1 µmol) (siehe Schritt 3.1) in Phosphat-gepufferter Kochsalzlösung (2 ml) wurde mit einer Suspension des gemäß Schritt 2 gewonnenen Produktgemisches (4 mg) in Dimethylformamid (400 µl) versetzt und 1 h bei Raumtemperatur inkubiert. Dann wurde das Reaktionsgemisch über eine Sephadex G15-Säule in Phosphat gepufferter Kochsalzlösung Gel-filtriert. Das proteinhaltige Eluat wurde gesammelt und bei -30° C präserviert (Ausbeute 4.2 mg).

Schritt 3.1:

Aminosäuresequenz des fos-Peptids (| |) mit N-terminaler GGC Verlängerung.

Ac-CGGyLTDTLQAETDQLEDKKSALQTEIANLLKEKEKLEFILAAYy Die Buchstaben stehen für folgende Aminosäuren: A = Alanin, C = Cystein, D = Asparaginsäure, E = Glutaminsäure, G = Glycin, I = Isoleucin, K = Lysin, L = Leucin, M = Methionin, N = Asparagin, Q = Glutamin, R = Arginin, S = Serin, T = Threonin, V = Valin, Y = Tyrosin.

Die Oligopeptidsynthese erfolgte mittels eines automatischen Peptid-Synthesizers (Applied Biosystems Model 430A) entsprechend der Merryfield Festphasen Methode (Stewart und Young. Solid Phase Synthesis. Pierce Chemical Company. 2. Edition. Rockford Ill.) mit der tert-butyloxycarbonyl Schutzgruppe. Die Oligopeptide wurden von dem Phenylacetamidomethylpolystyrene-Träger abgespalten. Nach Abspaltung der Schutzgruppen (Tom et al. 1983, J. Am. Chem. Soc. 105, 6442-6455) wurden die Oligopeptide über reversed phase Chromatographie (PepRPC-Säule, Pharmacia) wie bei Rivier et al. (J. Chromatography 288, 303-328, 1984) beschrieben gereinigt.

Schritt 4:

Herstellung eines fos-Peptid-EDTA-Yttrium-Chelats mit einem nach Schritt 3 hergestellten fos-peptid-EDTA-Konjugates

Das gemäß Schritt 3 hergestellte fos-Peptid-EDTA-Konjugat (4.2 mg) wurde gegen isotonische Kochsalzlösung/0.1 M Natriumcitrat, pH 7.0, in einem Dialyseschlauch mit Ausschlußgrenze, m.w. 1000 (Spectrum) dialysiert und mit 6 mg Yttriumchlorid, die in 3 ml isotonischer Kochsalzlösung/0.1 M Natriumcitrat, pH 7.0, gelöst waren, versetzt. Nach 1 h wurde gegen Phosphat-gepufferte Kochsalzlösung rückdialysiert und die Chelatlösung bei -30° C präserviert. Das im obigen Beispiel beschriebene fos-Peptid-EDTA-Yttrium-Chelat wird dann als Ligand benutzt, um mit hoher Avidität an den jun-Peptid-Arm des bi- oder oligospezifischen Makromoleküles zu binden. Die Konstruktion eines für diese Interaktion besonders gut geeigneten bispezifischen Makromoleküles ist im folgenden Beispiel beschrieben.

B) Konstruktion des MAk-jun Fusionsmoleküles

Die hier verwendeten Techniken wurden, wenn nicht anders angezeigt, aus Maniatis et al. (Laboratory Manual EMBL (1982), Heidelberg, und Sambrook (Molecular Cloning: A Laboratory Manual) entnommen.

Schritt 1:

Ein humanes IgG3 C-Gen wurde aus einer humanen Genbank in EMBL 3 Phagen isoliert (A.M. Frischauf et al., J. Mol. Biol. 170, 827-842, 1983 und G.H.A. Seemann et al., The EMBO Journal 5, 547-552, 1986). Aus diesem IgG3 C-Gen wurde, wie in der deutschen Patentanmeldung P 3825615.0 beschrieben, eine Konstruktion (D) hergestellt, die nur noch das CH1 Exon und das erste Hinge Exon des IgG3 C-Gens enthält (Fig. 14).

Aus der gleichen Genbank wurde, wie ebenfalls in der deutschen Patentanmeldung P 3825615.0 beschrieben, ein humanes HLA B27k Gen isoliert. Aus diesem HLA B27w Gen wurde ein Konstrukt (E) hergestellt, das nur noch das C3 Exon und den 3′ NT Bereich des HLA B27k Gens enthält (Fig. 15).

Schritt 2:

Das C1 Exon und der 3′ NT Bereich des HLA B27k Gens wurden mit Xba1 aus dem Plasmid E herausgeschnitten, das Fragment isoliert und in die Xba1 Schnittstelle des Konstruktes D kloniert. Durch Restriktionsanalyse und Nukleinsäuresequenzanalyse wurde der Klon F identifiziert, der das C3 Exon und den 3′ NT Bereich des HLA B27c Gens in der korrekten 5′-3′ Orientierung 3′ vom IgG3 C-Gen Fragment enthält (Fig. 16).

Schritt 3:

Das Insert des Klons F wird unter Verwendung der Endonukleasen Hind III und Eco RI aus dem Plasmid ausgeschnitten und zwischen die Hind III und Eco RI Schnittstellen eines M13mp18 Doppelstrang (DS)-Phagen kloniert. Es wird der Phagenklon G isoliert, der das Antikörper/HLA Fusiongenfragment enthält (Fig. 17).

Schritt 4:

Vom Phagenklon G werden nach der Methode von T.A. Kunkel, 1985, Proc. Natl. Acad. Science, U.S.A., 82, 488-492, Uracileinzelstränge präpariert. Die Einzelstrangphagen wurden mit den mutagenen Oligonukleotiden 1 und 2 (Tab. 6) hybridisiert und die Lücken zwischen den Oligonukleotiden mit Klenon DNA Polymerase und T4 Ligase geschlossen. Nach Transformation in E. coli wurde durch Restriktionsanalyse und Nukleinsäuresequenzanalyse ein Phagenklon identifiziert (G), bei dem die Sst1 Restriktionsschnittstelle am 5′ Ende des Hinge Exons deletiert wurde. Gleichzeitig wurden am 3′ Ende des Hinge Exons eine Sst1 und eine Sph1 Restriktionsschnittstelle eingeführt (Fig. 18). Zur Deletion der Sst1 Schnittstelle wurde die dritte Base des 2. Codons des Hinge Exons von C in G umgewandelt und für die Einführung der Sst1 und Sph1 Schnittstellen wurden zwischen dem 15. und 16. Codon des Hinge Exons die Basen 5′GAGCTCGGGGCA3′ eingeführt (Tab. 7).

Schritt 5:

Doppelstrang DNA des Phagenklons G′ wird mit Sph1 vollständig und mit Sst1 partiell gespalten. Die synthetischen Oligonukleotide Jun I und Jun II (Tab. 8) werden zu einem Doppelstrang DNA Fragment zusammengefügt, welches an seinen Enden je eine geschnittene Sph1 und Sst1 Restriktionsschnittstelle enthält und für ein Peptid kodiert, das den Jun Leucin Zipper enthält (O'Shea et al., Science, 245, 646-648, 1989).

Das doppelsträngige DNA Fragment wird in die Sst1 und Sph1 Restriktionsschnittstellen des F′ Phagenklons kloniert und der Phagenklon H identifiziert, der ein Genkonstrukt enthält, bei dem im Hinge Exon die Sequenz für das Jun Zipper Peptid inseriert ist (Fig. 19).

Schritt 6:

Das Insert des dS Phagen H wurde mit den Restriktionsendonukleasen Hind III und Eco RI ausgeschnitten, die Enden mit T4 Polymerase aufgefüllt und in einen Sma1 gespaltenen KsF Vektor (Stratagene, 11099 North Torrey Pines Road, La Jolla California 92037) kloniert. Es wurde der Plasmidklon I identifiziert, der das Antikörper/Jun/HLA Fusionsgen in der Orientierung enthält (Fig. 20), in der es auf beiden Seiten von einer BamH1 Schnittstelle flanklert wird.

Schritt 7:

Das Antikörper/Jun/HLA Fusionsgen wurde mit BamH1 aus dem KS Klon H1 ausgeschnitten und in das Expressionsplasmid pABStop (Behringwerke AG) kloniert, welches ein spezifisches funktionelles Immunglobulin V-Gen enthält. Das spezifische V-Gen wurde, wie in der Patentanmeldung P 3909799.4 beschrieben, gewonnen. Es wurde das Expressionsplasmid I identifiziert, das das Antikörper/Jun/HLA Fusionsgenkonstrukt in der korrekten Orientierung hinter dem $V_H$ Gen enthält (Fig. 21).

Die Kotransformation des Plasmids I mit einem Plasmid, der das Gen für die leichte Kette des spezifischen MAk enthält und einen Plasmid, der ein Resistenzgen trägt, führt zur Expression eines spezifischen Antikörper F(ab′)2 Fragmentes, das in der Hinge Region zwei Jun Zipper Peptide enthält, wobei das Jun Zipper Peptid so verändert ist, daß keine Homodimerbildung (Jun/Jun) mehr stattfindet.

Beispiel 6:

Optimierung der bi- oder oligospezifischen Rezeptormenge am Tumor und Minimierung derselben im Blut bzw. den Normalgeweben.

Wissenschaftliche Untersuchungen anderer haben gezeigt, daß die Penetration von soliden Tumoren mit Makromolekülen > 50 kDa langsam von statten geht und meist nur die Randregion bzw. einige wenige Areale im Tumor erreicht werden. Diese Untersuchungen basieren auf Experimenten, die eine einmalige Injektion niedriger Mengen von Makromolekülen beinhalten. Im Gegensatz hierzu haben wir gefunden, daß durch repetitive i.v.-Injektion großer Mengen von bi- oder oligospezifischen Rezeptoren (10 x 250 µg Rezeptor/Maus für 10 Tage) eine weitgehende Durchdringung der gesamten Tumormasse in Nacktmaus-Xenografts möglich ist. Des weiteren bleiben die bi- oder oligospezifischen Rezeptoren aufgrund ihrer spezifischen Bindung an TAA für lange Zeiträume (> 20 Tage) in großen

Mengen an der Tumorzellmembran und im Tumorinterstitium hängen. Diese Befunde wurden mittels der indirekten alkalischen Phosphatasetechnik an kryopräservierten Dünnschnitten von humanen Colon- und Pankreas-Tumor-Xenografts erhoben.

Während dieser Zeit (schon nach 10 Tagen) wurden die bi-oder oligospezifischen Rezeptormoleküle aus den TAA-negativen Normalgeweben und dem Blut durch Abbau und Ausscheidung bereits eliminiert. Um diesen Eliminationszeitraum zu verkürzen, wurde ein anti-idiotypischer MAk (anti Id), der nur mit dem anti TAA-Arm nicht gebundener bi- oder oligospezifischer Rezeptormoleküle reagiert, 24 Stunden nach Beendigung der 10 x Injektion von bi- oder oligospezifischen Rezeptoren i.v. injiziert (1 x50 µg anti Id). Diese einmalige Injektion bewirkte eine beschleunigte Elimination der ungebundenen bi- oder oligospezifischen Rezeptormoleküle aus dem Blut und eine schnellere Metabolisierung in Leber und Milz.

Aufgrund dieser Manipulation kann die Injektion des Komplexons (EDTA-Y90) schon 4 Tage nach Beendigung der Penetrations- und Bindungsphase des bi- oder oligospezifischen Rezeptors erfolgen. Aus diesen Untersuchungen leitet sich folgendes Behandlungsschema (für Nacktmäuse) ab:

a) Tag 1-10, i.v. Injektion von je 1x250 µg bi- oder oligospezifischem Rezeptor

b) Tag 11, i.v. Injektion von 1x50 µg anti Id

c) Tag 14, i.v. Injektion einer therapeutischeen Dosis von EDTA-Y90.

Aufgrund vergleichender immunszintigraphischer Daten in Nacktmäusen bzw. Tumorpatienten sollte dieses Schema auch für die Tumortherapie im Menschen geeignet sein. Allerdings bewegen sich die im Humansystem zu injizierenden Mengen in einer anderen Größenordnung. 10x 5-10 g bispez. Rezeptor, 1x1 g anti Id. Die Injektion des anti Id ist für die Therapie nicht zwingend.

Anlage 1a

Quantitative Inhibition-ELISA für MAk durch DTPA bzw. EDTA Komplexe

Material: Teilbare 96-well Polystyrol Mikrotiterplatten (U-Form) Typ B, Fa. Nunc, Nr. 4-60445

1) Pro well werden 50 µl Y Benzyl-DTPA-HSA 19-Konjugat mit einer Konz. von 1 µg Konjugat pro ml PBS, pH 7.2, pipettiert und über Nacht bei Raumtemperatur (RT) inkubiert.
2) Der Überstand wird abgesaugt und 3x mit 0.05 M Tris-Citrat- Puffer, pH 7.4, (Waschlsg. 1) gewaschen; (1x waschen = 250 µl Waschlösung pro well einfüllen, 2 min stehen lassen, absaugen)
3) Wenn die Mikrotiterplatte nicht direkt benötigt wird, wird sie über Nacht auf Zellstoff bei RT stehengelassen (Öffnung nach unten). Danach wird die Platte in Folien mit Trockenpatronen eingeschweißt (Fa. Gaplast, Postfach 529, 8100 Garmisch-Partenkirchen). Unter diesen Bedingungen sind die Platten bei +4° C mindestens 8 Wochen haltbar.
4) 250 µl Blocklösung werden pro well aufgetragen und 30 min bei 37° C inkubiert.
5) Während des Blockierens erfolgt die Vorinkubation des verdünnten Hybridomüberstandes mit dem Kompetitor (siehe Anlage 2).
6) Von den zu testenden, entsprechend vorverdünnten und vorinkubierten Hybridomaüberständen werden pro well 50 µl aufgetragen und 30 min bei RT inkubiert.
7) Anschließend wird 3x mit Waschlösung 2 gewaschen.
8) Anschließend werden 50 µl 1:500 in Blocklösung verdünnter, mit alkalischer Phosphatase markierter Ziege anti Maus $IgG_1$-Antikörper pro well aufgetragen und 30 min bei RT inkubiert.
9) Danach wird 3x mit Waschlösung für Enzygnost gewaschen.
10) Anschließend werden 50 µl 0,1 mM NADP zugegeben.
11) Danach wird 30 min bei RT inkubiert.
12) Während der Inkubation mit NADP wird das Verstärkersystem wie folgt angesetzt:
pro Platte werden zu 2 Teilen INT und 1 Teil PBS, pH 7.2, zugegeben, des weiteren 1 Teil Diaphorase, des weiteren wird 1 Teil ADH zupipettiert.
13) 50 µl dieses Verstärkersystems werden pro well zugegeben.
14) Bei deutlichem Farbumschlag von durchsichtig nach rot wird die Reaktion mit 100 µl einer 0,1 N $H_2SO_4$-Lösung pro well abgestoppt.
15) Die Extinktionen werden bei 492 nm im TITERTEK$^R$ MULTISCAN vermessen. Als Blankwert werden 50 µl NADP mit 50 µl Verstärkerlösung und 100 µl 0,1 N $H_2SO_4$ eingesetzt.

| | |
|---|---|
| NADP | - Fa. Sigma Best.Nr. N-0505 |
| INT | - Fa. Sigma Best.Nr. I-8377 |
| ADH | - Fa. Sigma Best.Nr. A-3263 |
| DIAPHORASE | - Fa. Sigma Best.Nr. D-2381 |
| Waschlösung 2 | - Fa. Behring, Best.Nr. OSEW96, enthält Tween/PBS |

Blocklösung:

PBS, pH 7.2, wird durch Zugabe von Casein und 30minütiges Einrühren 3%ig an Casein gemacht und auf pH 7.4 eingestellt. Danach werden Partikel 10′ bei 4000 U/min abzentrifugiert.

Verdünnter mit alkalischer Phosphatase markierter Ziege anti Maus $IgG_1$-Antikörper (Fa. Southern Biotechnology Associates, Cat.Nr. 1080-04)

Herstellung von 0.1 mM NADP:

7.65 mg NADP lösen in 100 ml 20 mM Tris, 0.1 mM $MgSO_4$, pH 9.5; die Lösung kann bei -20° C mehrere Monate gelagert werden.

Herstellung von INT (P-IODO NITROTETRAZOLIUM Violet):

2.5 mg/ml 30%igem Ethanol im Ultraschallbad lösen; immer frisch ansetzen.

Herstellung von Diaphorase:

1 mg Diaphorase/ml PBS, pH 7.2, wird portioniert bei -20° C gelagert.

Herstellung von Alkoholdehydrogenase:

0.5 mg ADH/ml PBS, pH 7.2, werden portioniert bei -20° C gelagert.

Anlage 1b

Vorinkubation des Hybridomüberstandes mit dem Kompetitor

Die Maus IgG Konzentrationsbestimmung in Hybridomüberständen kann mittels kommerziell erhältlicher quantitativer ELISA Testsysteme bestimmt werden und ist Stand der Technik.

Anhand der Konzentrationsbestimmung im ELISA werden die Hybridomüberstände auf 1.25 µg/ml in PBS ohne $Ca^{++}$ und $Mg^{++}$ verdünnt.

Umrechnung von Gramm in Mol:

150 000 g - 1 Mol MAk

$1.25 \times 10^{-6}$ g- xMol

$$1.25\ \mu g = x = 8.33\ 10^{-12}\ \text{Mol}$$

Um ein Verhältnis 1+1 von MAk und Inhibitor zu haben, wurden zu 50 µl Hybridomüberstand mit einer Konzentration von $8.33 \times 10^{-12}$ Mol/ml 10 µl Inhibitor mit einer um den Faktor 5 erhöhten Konzentration von $8.33 \times 10^{-12}$ Mol/200 µl gegebenen.

Der Hybridomüberstand wird mit 100 000fachem, 50 000fachem, 10 000fachem, 5 000 fachem, 1 000 fachem und 100fachem Kompetitorüberschuß für 30′ bei RT inkubiert. Hiervon werden 50 µl in den ELISA (siehe Anlage 1a, Punkt 6) pipettiert.

Anlage 1c

Erzeugung der DTPA- bzw. EDTA-Komplexe

Die Komplex-Konstante von DTPA bzw. EDTA zu den in Tabelle I dargestellten Metallionen ist extrem hoch, so daß bei äquimolarer Mischung von DTPA bzw. EDTA mit diesen Metallionen eine komplette Sättigung zu erwarten ist. Die entsprechenden Metallionen wurden deswegen in einem 3fachen molaren Überschuß mit den DTPA bzw. EDTA inkubiert. Als Beispiel wurden 170 µl einer 10 mM Cadmiumsulfatlösung in bidest (siehe Anlage 1d) mit 30 µl einer 0.028

molaren DTPA-Stammlösung in bidest für 5′ bei RT inkubiert. Zumischung von 10 µl dieser Kompetitorlösung zu dem Hybridomüberstand führt zu einem 100000fachen Überschuß an Kompetitor über den im Hybridomüberstand enthaltenen MAk. Niedrigere Kompetitor zu MAk Verhältnisse wurden dadurch erzielt, daß die Kompetitorlösung entsprechend dem gewünschten molaren Überschuß (siehe Anlage 1b) in der jeweiligen Salzionenlösung verdünnt wurde.

Anlage 1d

Quelle und relevante physicochemische Parameter der eingesetzten Metallionen

Von folgenden Metallionen wurden 10m molare Lösungen in bidest hergestellt:

| | | |
|---|---|---|
| Manganchlorid (?) | MG 161,88 | |
| Fa. Merck | Nr. 5934 | Ionenradius Mn: 80 pm |
| Cadmiumsulfate | MG 256,5 | |
| Riedel de Haen | Nr. 31145 | Ionenradius Cd: 97 pm |
| Zinkchlorid | MG 136,28 | |
| Fa. Merck | Nr. 8816 | Ionenradius Zn: 74 pm |
| Kupfersulfat | Mg 159,61 | |
| Fa. Riedel de Haen | Nr. 31294 | Ionenradius Cu: 96 pm |
| Yttriumchlorid | MG 303,36 | |
| Fa. Aldrich | Nr. 20,491-9 | Ionenradius Y: 92 pm |
| Blei(II)-nitrat | MG 331,20 | |
| Fa. Riedel de Haen | Nr. 31137 | Ionenradius Pb: 120 pm |

Anlage 1e

Quantitativer Inhibitionstest von MAk durch DTPA bzw. EDTA

molarer Überschuß an Kompetitor der zu 50 % Inhibition der Bindung an das Festphasenantigen führt.

| MAK-Nr. | DTPA-Y | DTPA | DTPA-Mn | DTPA-Cd | DTPA-Zn | DTPA-Cu |
|---|---|---|---|---|---|---|
| 2050/174 | $10^4$ | $10^3$ | $10^2$ | $10^2$ | $5x10^3$ | $5x10^3$ |
| 2050/531 | $5x10^4$ | $10^3$ | $10^2$ | $10^2$ | $5x10^3$ | $5x10^3$ |
| 2050/532 | $5x10^4$ | $10^3$ | $10^2$ | $10^2$ | $5x10^3$ | $5x10^3$ |
| 2050/534 | $5x10^4$ | $10^3$ | $10^2$ | $10^2$ | $5x10^3$ | $5x^{103}$ |
| 2050/535 | $10^4$ | $10^2$ | $10^2$ | $10^2$ | $10^3$ | $10^3$ |

| MAK-Nr. | DTPA-Pb | 1.2.Diaminoethan | Transaconitsäure | EDTA-Y | EDTA | EDTA-Mn |
|---|---|---|---|---|---|---|
| 2050/174 | $10^3$ | keine Inhibition bis $10^5$ | keine Inhibition bis $10^5$ | $10^2$ | $10^3$ | $10^3$ |
| 2050/531 | $5x10^3$ | " | " | $10^3$ | $10^3$ | $10^3$ |
| 2050/532 | $5x10^3$ | " | " | $10^2$ | $10^3$ | $10^3$ |
| 2050/534 | $5x10^3$ | " | " | $10^2$ | $10^3$ | $10^2$ |
| 2050/535 | $10^3$ | " | " | $10^2$ | $10^2$ | $10^2$ |

| MAK-Nr. | EDTA-Cd | EDTA-Zn | EDTA-Cu | EDTA-Pb |
|---|---|---|---|---|
| 2050/174 | $10^3$ | $10^3$ | $10^3$ | $5x10^3$ |
| 2050/531 | $10^3$ | $10^3$ | $10^2$ | $10^5$ |
| 2050/532 | $10^3$ | $10^3$ | $5x10^3$ | $10^5$ |
| 2050/534 | $10^2$ | $10^3$ | $10^3$ | $5x10^3$ |
| 2050/535 | $10^2$ | $10^2$ | $10^2$ | $10^2$ |

Tab. 1

Peptidlinker mit dazugehörigen Nukleotidsequenzen

```
              E  P  K  S  C  G  G  E  A  A  P  A
+ 5' CTCTCTGCAGAGCCCAAATCTTGTGGCGGCGAGGCAGCTCCCGCAG
- 3' GAGAGACGTCTCGGGTTTAGAACACCGCCGCTCCGTCGAGGGCGTC

   A  A  P  A  A  A  A  A  G  G  Q  V  Q  L  Q  E  S
CTGCACCCGCAGCAGCCGCAGCAGGCGGGCAGGTCCAACTGCAGGAGAGC 3'
GACGTGGGCGTCGTCGGCGTCGTCCGCCCGTCCAGGTTGACGTCCTCTCG 5'
```

+ = kodierender Strang

- = komplementärer Strang

Tab. 2 MAK A

**VH**

```
 Q  V  Q  L  Q  E  S  G  G  G  L  V  Q  P  G  G  S  L  R  L
CAGGTCCAACTGCAGGAGTCTGGAGGAGGCTTGGTACAGCCTGGGGGTTCTCTGAGACTC
         10        20        30        40        50        60

 S  C  A  T  S  G  F  S  D  Y  Y  M  N  W  V  R  Q  P  P  G
TCCTGCGCAACTTCTGGGTTCAGTGATTACTACATGAACTGGGTCCGCCAGCCTCCAGGA
         70        80        90       100       110       120

 K  A  L  E  W  L  G  F  I  S  N  K  P  N  G  H  T  T  E  Y
AAAGCACTTGAGTGGTTGGGTTTTATTTCAAACAAACCTAATGGTCACACAACAGAGTAC
        130       140       150       160       170       180

 S  A  S  V  K  G  R  F  T  I  S  R  D  N  S  Q  S  I  L  Y
AGTGCATCTGTGAAGGGTCGGTTCACCATCTCCAGAGATAATTCCCAAAGCATCCTCTAT
        190       200       210       220       230       240

 L  Q  M  N  T  L  R  A  E  D  S  A  T  Y  Y  C  A  R  D  K
CTTCAAATGAACACCCTGAGAGCTGAGGACAGTGCCACTTATTATTGTGCAAGAGATAAG
        250       260       270       280       290       300

 G  I  R  W  Y  F  D  V  W  G  Q  G  T  T  V  T  V  S  S
GGAATACGATGGTACTTCGATGTCTGGGGCCAAGGGACCACGGTCACCGTCTCCTCA
        310       320       330       340       350
```

**VK**

```
  A  I  L  S  A  S  P  G  E  K  V  T  M  T  C  R  A  S  S  S
AGCAATCCTGTCTGCATCTCCAGGGGAGAAGGTCACAATGACTTGCAGGGCCAGCTCAAG
         10        20        30        40        50        60

  V  S  Y  M  H  W  Y  Q  Q  K  P  G  S  S  P  K  P  W  I  Y
TGTAAGTTACATGCACTGGTACCAGCAGAAGCCAGGATCCTCCCCCAAACCCTGGATTTA
         70        80        90       100       110       120

  A  T  S  N  L  A  S  G  V  P  A  R  F  S  G  S  G  S  G  T
TGCCACATCCAACCTGGCTTCTGGAGTCCCTGCTCGCTTCAGTGGCAGTGGGTCTGGGAC
        130       140       150       160       170       180

  S  Y  S  L  T  I  I  R  V  E  A  E  D  A  A  T  Y  Y  C  Q
CTCTTACTCTCTCACAATCATCAGAGTGGAGGCTGAAGATGCTGCCACTTATTACTGCCA
        190       200       210       220       230       240

  Q  W  S  S  N  P  L  T  F  G  A  G  T  K  L  E  I
GCAGTGGAGTAGTAACCCGCTCACGTTCGGTGCTGGGACCAAGCTGGAGATC
        250       260       270       280       290
```

Tab. 3 MAK B

**VH**

```
 L  Q  E  S  G  P  D  L  V  K  P  S  Q  S  L  S  L  T  C  T
CTGCAGGAGTCAGGACCTGACCTGGTGAAACCTTCTCAGTCACTTTCACTCACCTGCACT
        10        20        30        40        50        60

 V  T  G  Y  S  I  T  S  G  Y  S  W  H  W  I  R  Q  F  P  G
GTCACTGGCTACTCCATCACCAGTGGTTATAGCTGGCACTGGATCCGGCAGTTTCCAGGA
        70        80        90       100       110       120

 N  K  L  E  W  M  G  Y  I  Q  Y  S  G  I  T  N  Y  N  P  S
AACAAACTGGAATGGATGGGCTACATACAGTACAGTGGTATCACTAACTACAACCCCTCT
       130       140       150       160       170       180

 L  K  S  R  I  S  I  T  R  D  T  S  K  N  Q  F  F  L  Q  L
CTCAAAAGTCGAATCTCTATCACTCGAGACACATCCAAGAACCAGTTCTTCCTGCAGTTG
       190       200       210       220       230       240

 N  S  V  T  T  E  D  T  A  T  Y  Y  C  A  R  E  D  Y  D  Y
AATTCAGTGACTACTGAGGACACAGCCACATATTACTGTGCAAGAGAAGACTATGATTAC
       250       260       270       280       290       300

 H  W  Y  F  D  V  W  G  A  G  T  T  V  T  V  S  S
CACTGGTACTTCGATGTCTGGGGCGCAGGGACCACGGTCACCGTCTCCTCA
       310       320       330       340       350
```

**VK**

```
 L  T  Q  S  P  A  I  M  S  A  S  L  G  E  E  I  T  L  T  C
CTGACCCAGTCTCCAGCAATCATGTCTGCATCTCTAGGGGAGGAGATCACCCTAACCTGC
        10        20        30        40        50        60

 S  T  S  S  S  V  S  Y  M  H  W  Y  Q  Q  K  S  G  T  S  P
AGTACCAGCTCGAGTGTAAGTTACATGCACTGGTACCAGCAGAAGTCAGGCACTTCTCCC
        70        80        90       100       110       120

 K  L  L  I  Y  S  T  S  N  L  A  S  G  V  P  S  R  F  S  G
AAACTCTTGATTTATAGCACATCCAACCTGGCTTCTGGAGTCCCTTCTCGCTTCAGTGGC
       130       140       150       160       170       180

 S  G  S  G  T  F  Y  S  L  T  I  S  S  V  E  A  E  D  A  A
AGTGGGTCTGGGACCTTTTATTCTCTCACAATCAGCAGTGTGGAGGCTGAAGATGCTGCC
       190       200       210       220       230       240

 D  Y  Y  C  H  Q  W  S  S  Y  P  T  F  G  G  G  T  K  L  E
GATTATTACTGCCATCAGTGGAGTAGTTATCCCACGTTCGGAGGGGGGACCAAGCTGGAG
       250       260       270       280       290       300
```

Tab. 4 MAK C

**VH**

```
Q  V  Q  L  Q  Q  S  G  P  E  L  V  K  P  G  A  S  V  K  M
CAGGTCCAACTGCAGCAGTCTGGACCTGAGCTGGTAAAGCCTGGGGCTTCAGTGAAGATG
        10        20        30        40        50        60

S  C  K  A  S  G  Y  T  F  T  Y  Y  V  I  H  W  V  K  Q  K
TCCTGCAAGGCTTCTGGATACACATTCACTTACTATGTTATTCACTGGGTGAAACAGAAG
        70        80        90       100       110       120

P  G  Q  G  L  E  W  I  G  Y  I  H  P  Y  N  A  G  T  E  Y
CCTGGGCAGGGCCTTGAGTGGATTGGATACATTCATCCTTACAATGCTGGTACTGAGTAC
       130       140       150       160       170       180

N  E  K  F  K  G  K  A  T  L  T  S  D  K  S  S  S  T  A  Y
AATGAGAAGTTCAAAGGCAAGGCCACACTGACTTCAGACAAATCCTCCAGCACAGCCTAC
       190       200       210       220       230       240

M  E  L  S  S  L  T  S  E  D  S  A  V  Y  Y  C  S  M  G  R
ATGGAGCTCAGCAGCCTGACCTCTGAGGACTCTGCGGTCTATTACTGTTCAATGGGACGA
       250       260       270       280       290       300

G  G  D  Y  W  G  Q  G  T  T  V  T  V  S  S
GGGGGTGACTACTGGGGCCAAGGGACCACGGTCACCGTCTCCTCA
       310       320       330       340
```

**VK**

```
L  T  Q  S  P  A  I  M  S  A  S  P  G  E  K  V  T  M  T  C
CTGACCCAGTCTCCAGCAATTATGTCTGCATCTCCTGGGGAGAAGGTCACCATGACCTGC
        10        20        30        40        50        60

S  A  S  S  S  V  S  Y  M  H  W  Y  Q  Q  K  S  G  T  S  P
AGTGCCAGCTCAAGTGTAAGTTACATGCACTGGTACCAGCAGAAGTCAGGCACCTCCCCC
        70        80        90       100       110       120

K  R  W  I  Y  D  T  S  K  L  A  S  G  V  P  A  R  F  S  G
AAAAGATGGATTTATGACACATCCAAACTGGCTTCTGGAGTCCCTGCTCGCTTCAGTGGC
       130       140       150       160       170       180

S  G  S  G  T  S  Y  S  L  T  I  S  S  M  E  A  E  D  A  A
AGTGGGTCTGGGACCTCTTACTCTCTCACAATCAGCAGCATGGAGGCTGAAGATGCTGCC
       190       200       210       220       230       240

T  Y  Y  C  Q  Q  W  S  S  N  P  F  T  F  G  A  G  T  K  L
ACTTATTACTGCCAGCAGTGGAGTAGTAACCCATTCACGTTCGGCGCGGGGACCAAGCTG
       250       260       270       280       290       300
```

Tab. 5 MAK D

**VH**

```
 A  E  S  G  P  G  L  V  R  L  T  S  L  S  I  T  C  T  V  S
GCAGAGTCAGGGCCTGGCCTGGTGCGCCTCACGAGCCTGTCCATCACTTGCACTGTCTCT
         10        20        30        40        50        60

 G  F  S  L  I  S  Y  G  V  H  W  V  R  Q  P  P  G  K  G  L
GGCTTTTCATTAATTAGTTATGGTGTACACTGGGTTCGCCAGCCTCCAGGAAAGGGTCTG
         70        80        90       100       110       120

 E  W  L  G  V  I  W  A  G  G  S  T  N  Y  N  S  A  L  M  S
GAGTGGCTGGGAGTAATATGGGCAGGTGGAAGCACAAATTATAATTCGGCTCTCATGTCC
        130       140       150       160       170       180

 R  L  S  I  S  K  D  N  S  K  S  Q  V  F  L  K  M  N  S  L
AGACTGAGCATCAGCAAAGACAACTCCAAGAGCCAAGTTTTCTTAAAAATGAACAGTCTG
        190       200       210       220       230       240

 Q  T  G  D  T  A  I  Y  Y  C  A  R  G  G  D  D  Y  D  G  F
CAAACTGGTGACACAGCCATATACTACTGTGCCAGAGGGGGGGATGATTACGATGGGTTT
        250       260       270       280       290       300

 A  Y  W  G  Q  G  T  T  V  T  V  S  S  G  E  S
GCTTACTGGGGCCAAGGGACCACGGTCACCGTCTCCTCAGGTGAGTCC
        310       320       330       340
```

**VK**

```
 L  T  Q  S  P  S  S  L  A  V  S  A  G  E  K  V  T  M  S  C
CTGACCCAGTCTCCATCCTCCCTGGCTGTGTCAGCAGGAGAGAAGGTCACTATGAGCTGC
         10        20        30        40        50        60

 K  S  S  Q  S  L  L  S  S  T  K  R  K  N  Y  L  A  W  Y  Q
AAATCCAGTCAGAGTCTGCTCAGCAGTACAAAGCGAAAGAACTACTTGGCTTGGTACCAG
         70        80        90       100       110       120

 Q  K  P  G  Q  S  P  K  L  L  I  Y  W  A  S  T  R  E  S  G
CAGAAACCAGGTCAGTCTCCTAAACTACTGATCTACTGGGCATCCACTCGGGAATCTGGG
        130       140       150       160       170       180

  Y  P  D  R  F  T  G  S  G  S  G  T  D  F  T  L  T  I  S  S
GTCCCTGATCGCTTCACAGGCAGTGGATCTGGGACAGATTTCACTCTCACCATCAGCAGT
        190       200       210       220       230       240

 V  Q  A  E  D  L  A  V  Y  Y  C  K  Q  S  Y  N  L  R  A  F
GTGCAGGCTGAAGACCTGGCAGTTTATTACTGCAAACAATCTTATAATCTTCGGGCGTTC
        250       260       270       280       290       300

 G  G  G  T  K  L  E  I  K
GGTGGAGGGACCAAGCTGGAGATCAAA
        310       320
```

Tab. 6

Mutagene Oligonukleotide:

1) 5'CTTACCTGGG.CATGCCCCGA.GCTCCCGTGG.GCATGT3'

2) 5'AGTGGGGTTT.TCAGCTCTGCAGAG3'

Tabelle 7

Mutiertes Hinge Exon:

E L K T P L G D T T H T C

AGAGCTCAAA.ACCCCACTTG.GTGACACAAC.TCACACATGC

G

P R C P

CCACGGTGCCCAGGT

GAGCTCGGGGCA

Sst1 Sph1

Tab. 8

Jun I Oligonukleotid

5'CTACGCTCGG.CTAGAGGAAA.AAGTGAAAAC.
CTTGAAAGCG.CAAAACTCCG.AGCTGGCATC.
CACGGCCAAC.ATGCTCAGGG.AACAGGTGGC.
ACAGCTTAAG.CAGAAAGTCA.TGAACCACCG.
ACCTGCATB3'.

Jun II Oligonukleotid

5'CAGGTCGGTG.GTTCATGACT.TTCTGCTTAA.
GCTGTGCCAC.CTGTTCCCTG.AGCATGTTGG.
CCGTGGATGC.CAGCTCGGAG.TTTTGCGCTT.
TCAAGGTTTT.CACTTTTTCC.TCTAGCCGAG.
CGATGAGCT3'.

Formel 1

DTPA

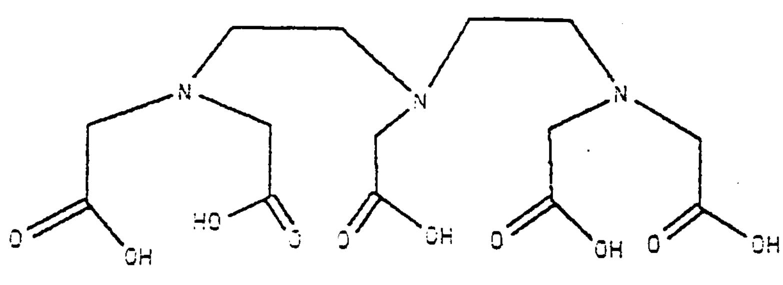

Formel 2

Isothiocyanatobenzyl-DTPA

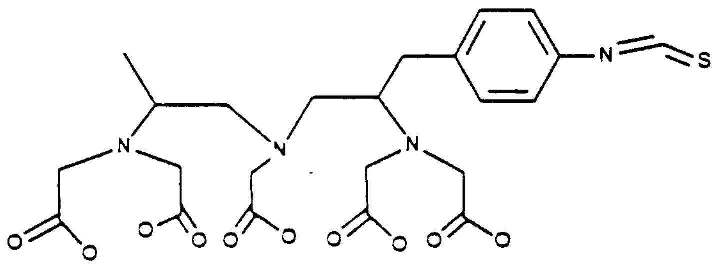

## Patentansprüche

**Patentansprüche für folgende Vertragsstaaten : AT, BE, CH, DE, DK, FR, GB, IT, LI, LU, NL, SE**

1. Bispezifische oder oligospezifische mono- oder oligovalente Rezeptoren, gentechnisch erhältlich durch Fusion von für $V_H$ und $C_H1$ Regionen der Antikörper zweier oder mehrerer verschiedener Spezifitäten kodierende DNA mittels geeigneter Linker und anschließende Expression zusammen mit den dazugehörenden Genabschnitten für die leichten Ketten in Expressionssystemen, dadurch gekennzeichnet, daß eine Spezifität über fos-jun Interaktion gegen ein Komplexon gerichtet ist.

2. Rezeptoren nach Anspruch 1, dadurch gekennzeichnet, daß die andere Spezifität gegen animale oder humane tumorassoziierte Antigene gerichtet ist.

3. Rezeptoren nach Anspruch 1 oder 2, dadurch gekennzeichnet, daß die andere Spezifität von den monoklonalen Antikörpern mit den variablen Regionen gemäß Tab. 2, 3, 4 oder 5 stammt.

4. Rezeptoren nach einem der Ansprüche 1 bis 3, dadurch gekennzeichnet, daß bei zwei Spezifitäten drei Bindungsstellen vorhanden sind.

5. Rezeptoren nach einem der Ansprüche 1 bis 3, dadurch gekennzeichnet, daß bei zwei Spezifitäten 4 Bindungsstellen vorhanden sind.

6. Rezeptoren nach einem der Ansprüche 1 bis 5, dadurch gekennzeichnet, daß bei drei Spezifitäten eine gegen Tumore gerichtet ist und die beiden anderen in unterschiedlicher Weise gegen DTPA oder EDTA gerichtet sind.

7. Verfahren zur Herstellung von Rezeptoren nach einem der Ansprüche 1 bis 6, dadurch gekennzeichnet, daß die für die schweren Ketten-Antikörperteile kodierenden DNA-Fragmente mittels geeigneter Linker verbunden und in einem Expressionssystem zusammen mit den Genen für die leichten Ketten exprimiert werden.

8. Arzneimittel enthaltend einen Rezeptor nach einem der Ansprüche 1 bis 6.

**Patentansprüche für folgende Vertragsstaaten : ES, GR**

1. Verfahren zur Herstellung von bispezifischen oder oligospezifischen mono- oder oligovalenten Rezeptoren wobei eine Spezifität über fos-jun Interaktion gegen ein Komplexon gerichtet ist, dadurch gekennzeichnet, daß für $V_H$ und

$C_H1$ Regionen der Antikörper zweier oder mehrerer verschiedener Spezifitäten kodierende DNA mittels geeigneter Linker fusioniert und anschließend zusammen mit den dazugehörenden Genabschnitten für die leichten Ketten exprimiert wird.

2. Verfahren nach Anspruch 1, dadurch gekennzeichnet, daß die andere Spezifität gegen animale oder humane tumorassoziierte Antigene gerichtet ist.

3. Verfahren nach Anspruch 1 oder 2, dadurch gekennzeichnet, daß die andere Spezifität von den monoklonalen Antikörpern mit den variablen Regionen gemäß Tab. 2, 3, 4 oder 5 stammt.

4. Verfahren nach einem der Ansprüche 1 bis 3, dadurch gekennzeichnet, daß bei zwei Spezifitäten drei Bindungsstellen vorhanden sind.

5. Verfahren nach einem der Ansprüche 1 bis 3, dadurch gekennzeichnet, daß bei zwei Spezifitäten 4 Bindungsstellen vorhanden sind.

6. Verfahren nach einem der Ansprüche 1 bis 5, dadurch gekennzeichnet, daß bei drei Spezifitäten eine gegen Tumore gerichtet ist und die beiden anderen in unterschiedlicher Weise gegen DTPA oder EDTA gerichtet sind.

## Claims

**Claims for the following Contracting States : AT, BE, CH, DE, DK, FR, GB, IT, LI, LU, NL, SE**

1. A bispecific or oligospecific mono- or oligovalent receptor, obtainable by gene manipulation by fusion of DNA coding for $V_H$ and $C_H1$ regions of the anti-bodies of two or more different specificities, by means of suitable linkers and subsequent expression together with the gene sections belonging thereto for the light chains in expression systems, wherein one specificity is directed against a complexone via fos-jun interaction.

2. The receptor as claimed in claim 1, wherein the other specificity is directed against animal or human tumor-associated antigens.

3. The receptor as claimed in claim 1 or 2, wherein the other specificity derives from the monoclonal anti-bodies with the variable regions shown in Tab. 2, 3, 4 or 5.

4. The receptor as claimed in any of claims 1 to 3, wherein three binding sites are present in the case of two specificities.

5. The receptor as claimed in any of claims 1 to 3, wherein 4 binding sites are present in the case of two specificities.

6. The receptor as claimed in any of claims 1 to 5, wherein, in the case of three specificities, one is directed against tumors and the two others are directed in a different manner against DTPA or EDTA.

7. A process for the preparation of the receptor as claimed in any of claims 1 to 6, which comprises the DNA fragments which code for the heavy chain anti-body portions being connected by means of suitable linkers and expressed in an expression system together with the genes for the light chains.

8. A pharmaceutical comprising the receptor as claimed in any of claims 1 to 6.

**Claims for the following Contracting States : ES, GR**

1. A process for the preparation of a bispecific or oligospecific mono- or oligovalent receptor, one specificity being directed against a complexone via fos-jun interaction, which comprises DNA coding for $V_H$ and $C_H1$ regions of the antibodies of two or more different specificities being fused by means of suitable linkers and subsequently expressed together with the gene sections belonging thereto for the light chains.

2. The process as claimed in claim 1, wherein the other specificity is directed against animal or human tumor-associated antigens.

3. The process as claimed in claim 1 or 2, wherein the other specificity derives from the monoclonal anti-bodies with the variable regions shown in Tab. 2, 3, 4 or 5.

4. The process as claimed in any of claims 1 to 3, wherein three binding sites are present in the case of two specificities.

5. The process as claimed in any of claims 1 to 3, wherein 4 binding sites are present in the case of two specificities.

6. The process as claimed in any of claims 1 to 5, wherein, in the case of three specificities, one is directed against tumors and the two others are directed in a different manner against DTPA or EDTA.

**Revendications**

**Revendications pour les Etats contractants suivants : AT, BE, CH, DE, DK, FR, GB, IT, LI, LU, NL, SE**

1. Récepteurs mono- ou oligovalents, bispécifiques ou oligospécifiques, pouvant être obtenus, par génie génétique, par fusion d'ADN codant pour les régions $V_H$ et $C_H1$ des anticorps à deux spécificités différentes ou plus, au moyen de lieurs appropriés, et expression subséquente avec les segments de gènes correspondants codant pour les chaînes légères, dans des systèmes d'expression, caractérisés en ce qu'une spécificité est dirigée contre un complexon par l'intermédiaire d'interaction *fos-jun*.

2. Récepteurs selon la revendication 1, caractérisés en ce que l'autre spécificité est dirigée contre des anti-gènes humains ou animaux associés à des tumeurs.

3. Récepteurs selon la revendication 1 ou 2, caractérisés en ce que l'autre spécificité dérive des anticorps monoclonaux comportant les régions variables selon le tableau 2, 3, 4 ou 5.

4. Récepteurs selon l'une des revendications 1 à 3, caractérisés en ce que, dans le cas de deux spécificités, trois sites de liaison sont présents.

5. Récepteurs selon l'une des revendications 1 à 3, caractérisés en ce que, dans le cas de deux spécificités, quatre sites de liaison sont présents.

6. Récepteurs selon l'une des revendications 1 à 5, caractérisés en ce que, dans le cas de trois spécificités, l'une est dirigé contre des tumeurs et les deux autres sont dirigées, de façon différente, contre le DPTA ou l'EDTA.

7. Procédé pour la production de récepteurs selon l'une des revendications 1 à 6, caractérisé en ce que les fragments d'ADN codant pour les segments d'anticorps de type chaîne lourde sont assemblés au moyen de lieurs appropriés et exprimés dans un système d'expression, conjointement avec les gènes codant pour les chaînes légères.

8. Médicament contenant un récepteur selon l'une des revendications 1 à 6.

**Revendications pour les Etats contractants suivants : ES, GR**

1. Procédé pour la production de récepteurs mono- ou oligovalents, bispécifiques ou oligospécifiques, une spécificité étant dirigée contre un complexon, par l'intermédiaire d'interaction *fos-jun*, caractérisé en ce que de l'ADN codant pour les régions $V_H$ et $C_H1$ des anticorps à deux spécificités différentes ou plus sont fusionnés au moyen de lieurs appropriés et ensuite exprimés conjointement avec les segments de gènes correspondants, codant pour les chaînes légères.

2. Procédé selon la revendication 1, caractérisé en ce que l'autre spécificité est dirigée contre des antigènes humains ou animaux associés à des tumeurs.

3. Procédé selon la revendication 1 ou 2, caractérisé en ce que l'autre spécificité dérive des anticorps monoclonaux comportant les régions variables selon le tableau 2, 3, 4 ou 5.

4. Procédé selon l'une des revendications 1 à 3, caractérisé en ce que, dans le cas de deux spécificités, trois sites de liaison sont présents.

5. Procédé selon l'une des revendications 1 à 3, caractérisé en ce que, dans le cas de deux spécificités, quatre sites de liaison sont présents.

6. Procédé selon l'une des revendications 1 à 5, caractérisé en ce que, dans le cas de trois spécificités, l'une est dirigé contre des tumeurs et les deux autres sont dirigées, de façon différente, contre le DPTA ou l,EDTA.

FIG. 1

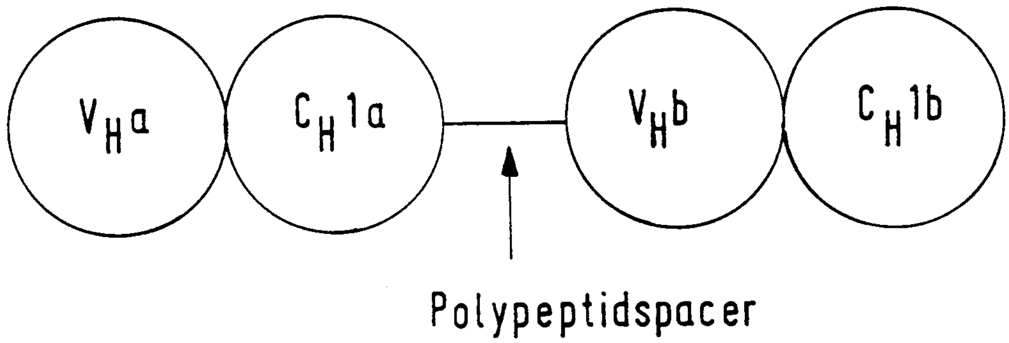
$V_H$a
$C_H$1a
$V_H$b
$C_H$1b
Polypeptidspacer

FIG. 2

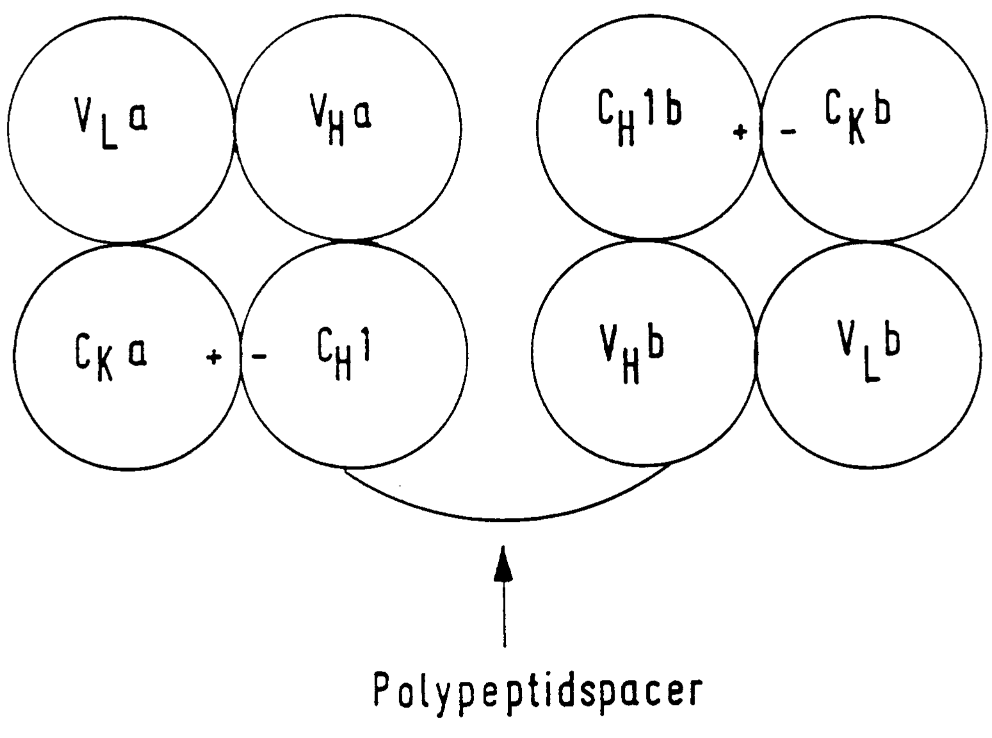
$V_L$a
$V_H$a
$C_H$1b
+
-
$C_K$b
$C_K$a
+
-
$C_H$1
$V_H$b
$V_L$b
Polypeptidspacer

FIG. 3

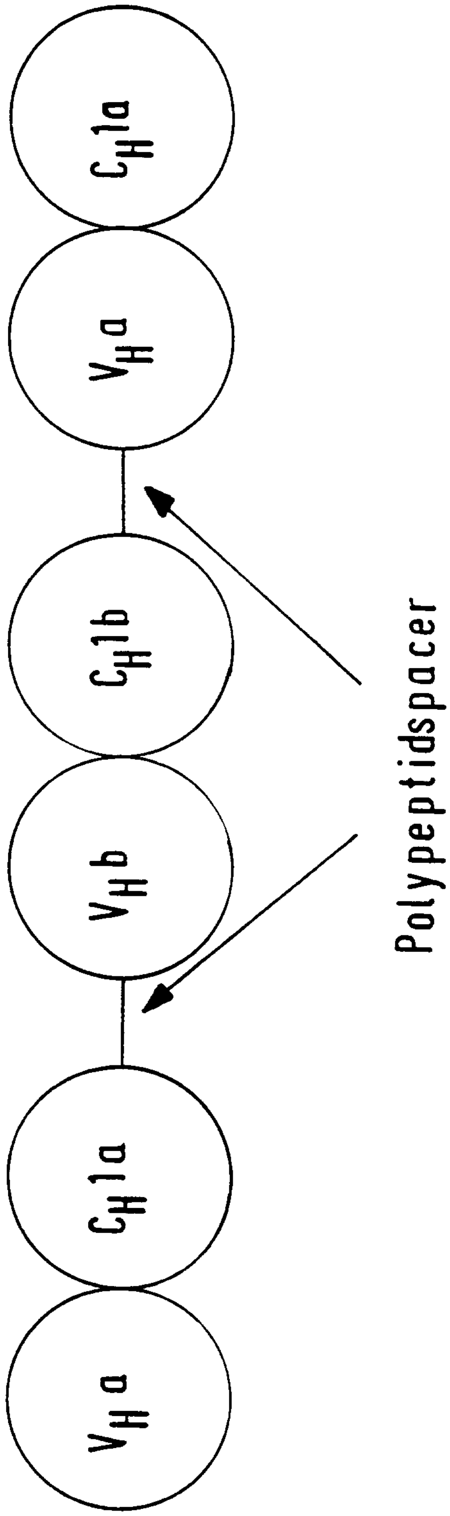

FIG. 4

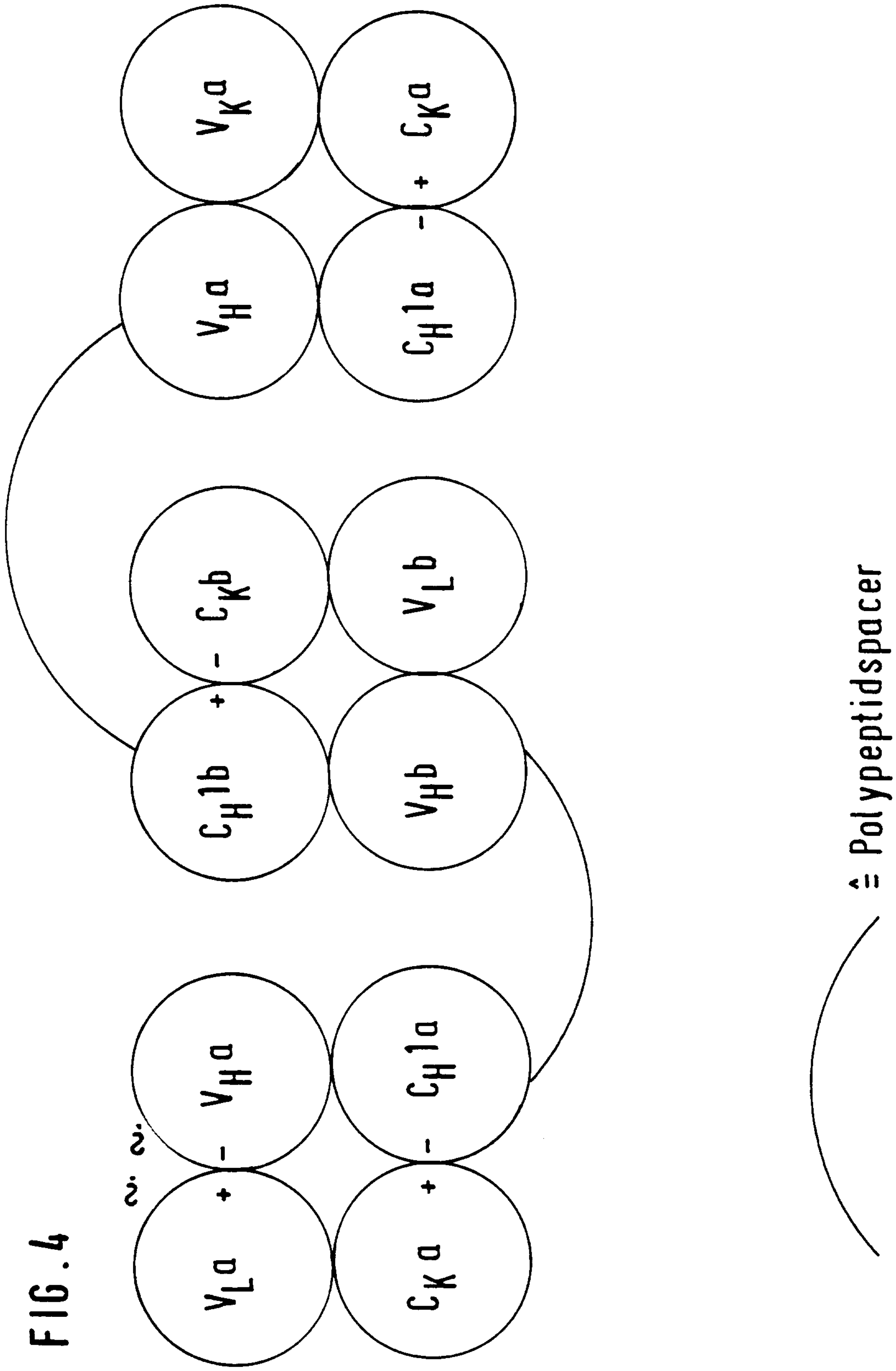
V_La
C_K a
V_Ha
C_H1a
C_H1b
C_Kb
V_Hb
V_Lb
V_Ha
C_H1a
V_Ka
C_Ka
≙ Polypeptidspacer

FIG. 5

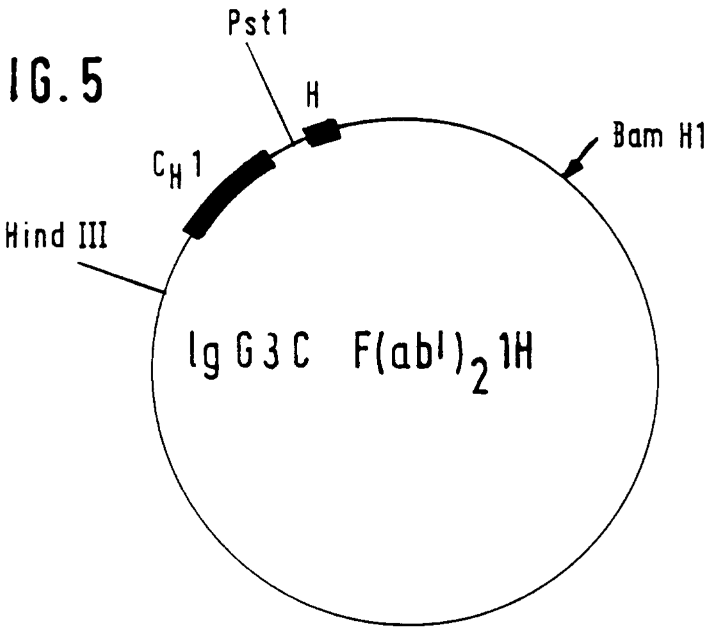
Pst1
H
Bam H1
$C_H$1
Hind III
Ig G3C F(ab')$_2$1H

FIG. 6

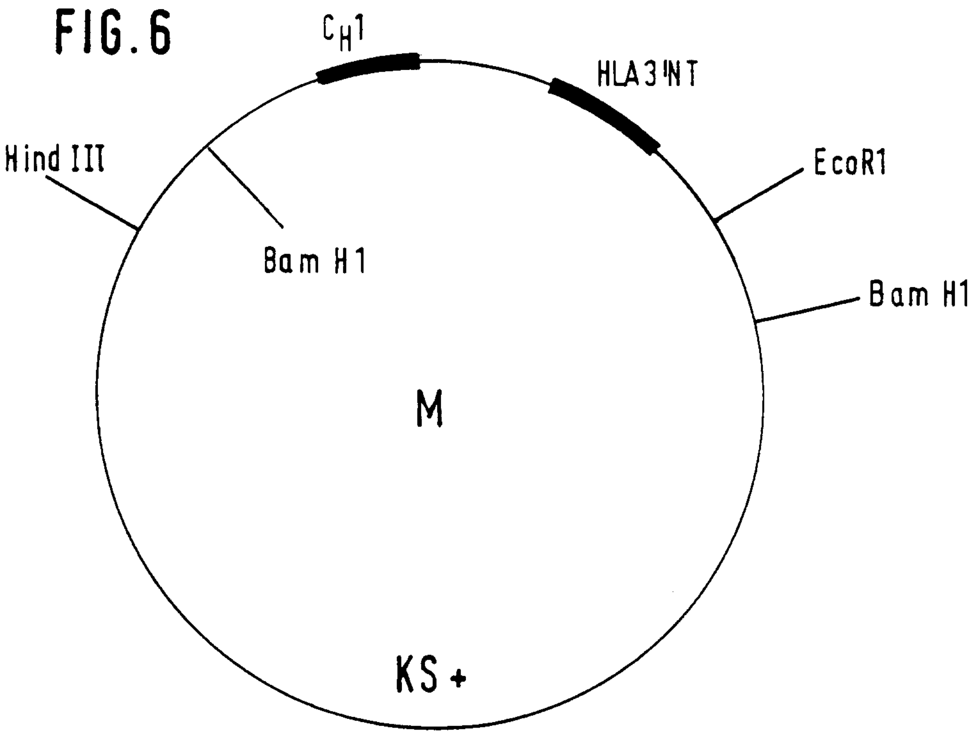
$C_H$1
HLA3'NT
Hind III
EcoR1
Bam H1
Bam H1
M
KS+

FIG.7

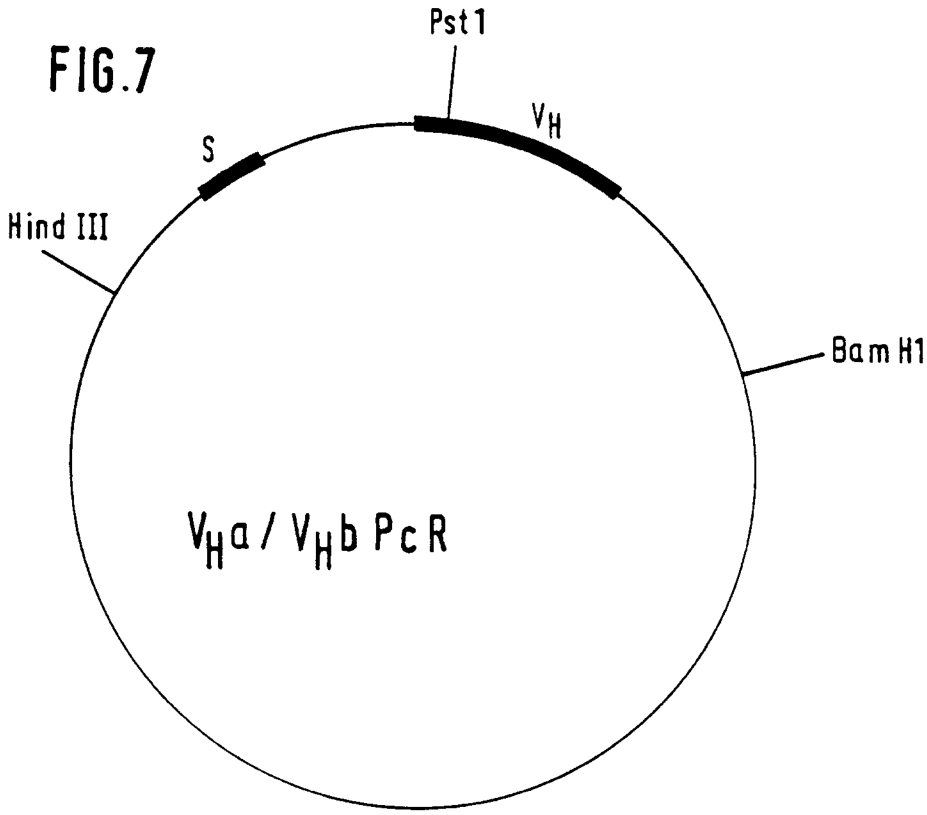
Pst1
$V_H$
S
Hind III
Bam H1
$V_Ha$ / $V_Hb$ PcR

FIG.8

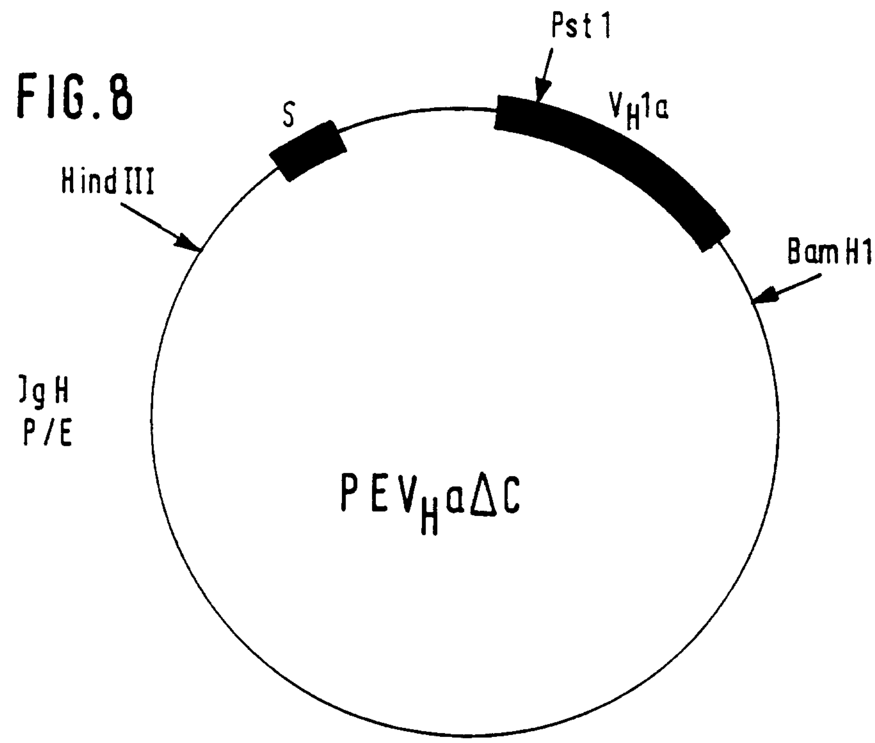
Pst1
$V_H1a$
S
Hind III
Bam H1
JgH
P/E
$PEV_Ha\Delta C$

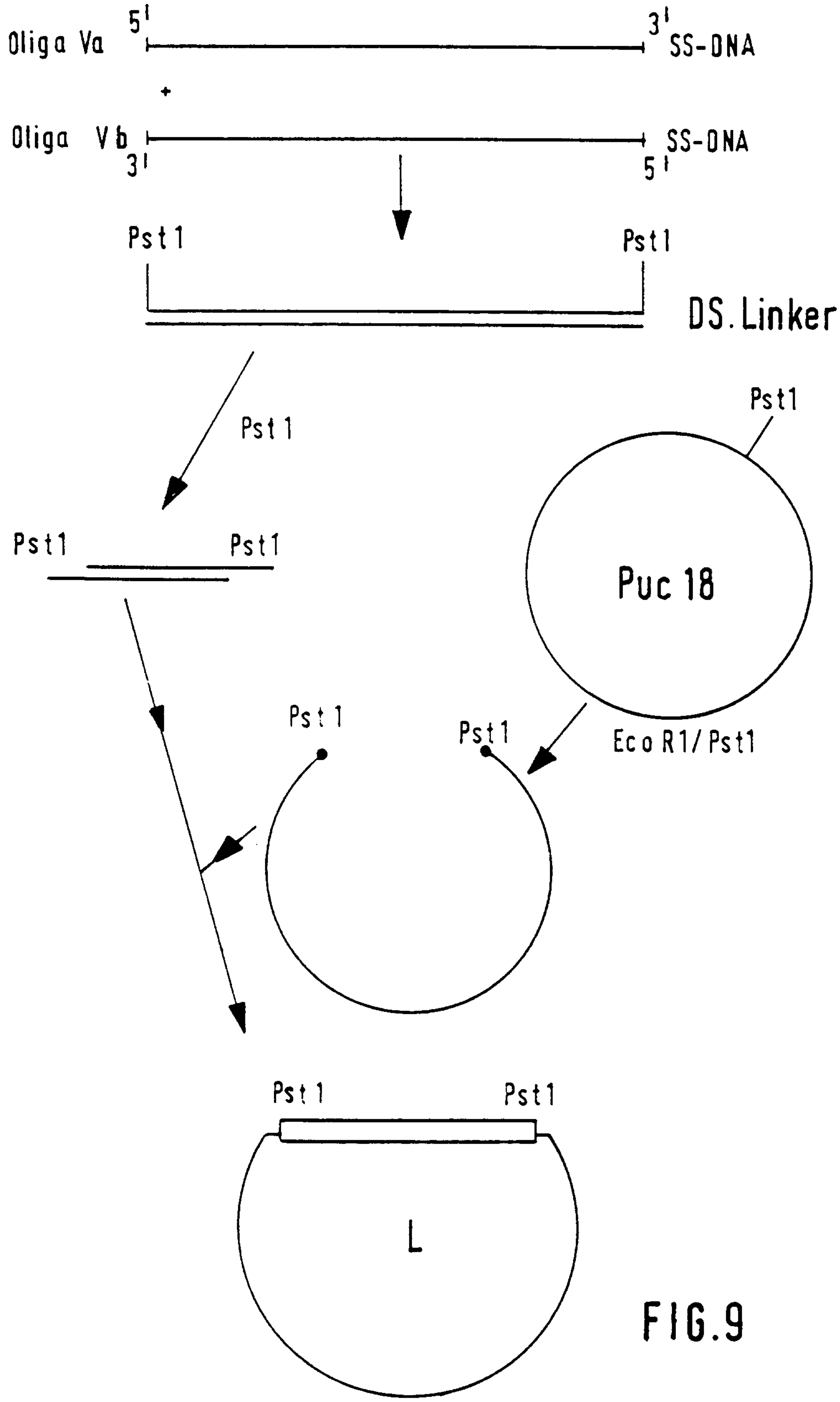
Oliga Va
5'
3'
SS-DNA
+
Oliga Vb
3'
5'
SS-DNA
Pst1
Pst1
DS.Linker
Pst1
Pst1
Puc 18
Pst1
Pst1
Pst1
Pst1
Eco R1/Pst1
Pst1
Pst1
L

FIG.9

FIG. 10

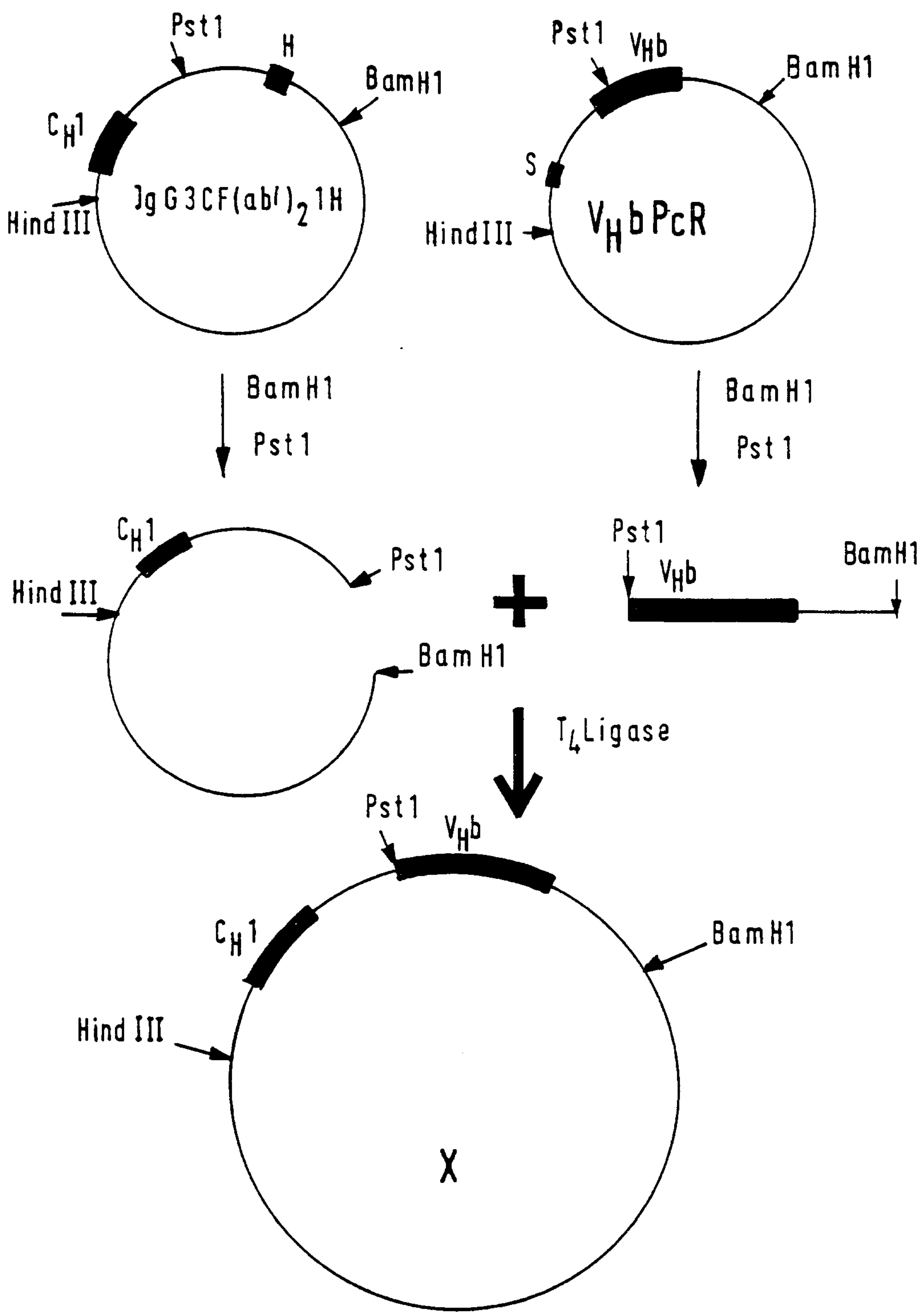
Pst1
H
BamH1
CH1
Hind III
Jg G3CF(ab')21H
Pst1
VHb
Bam H1
S
HindIII
VHbPcR
BamH1
Pst1
BamH1
Pst1
CH1
Pst1
Hind III
Bam H1
Pst1
VHb
BamH1
T4Ligase
Pst1
VHb
CH1
BamH1
Hind III
X

FIG.11

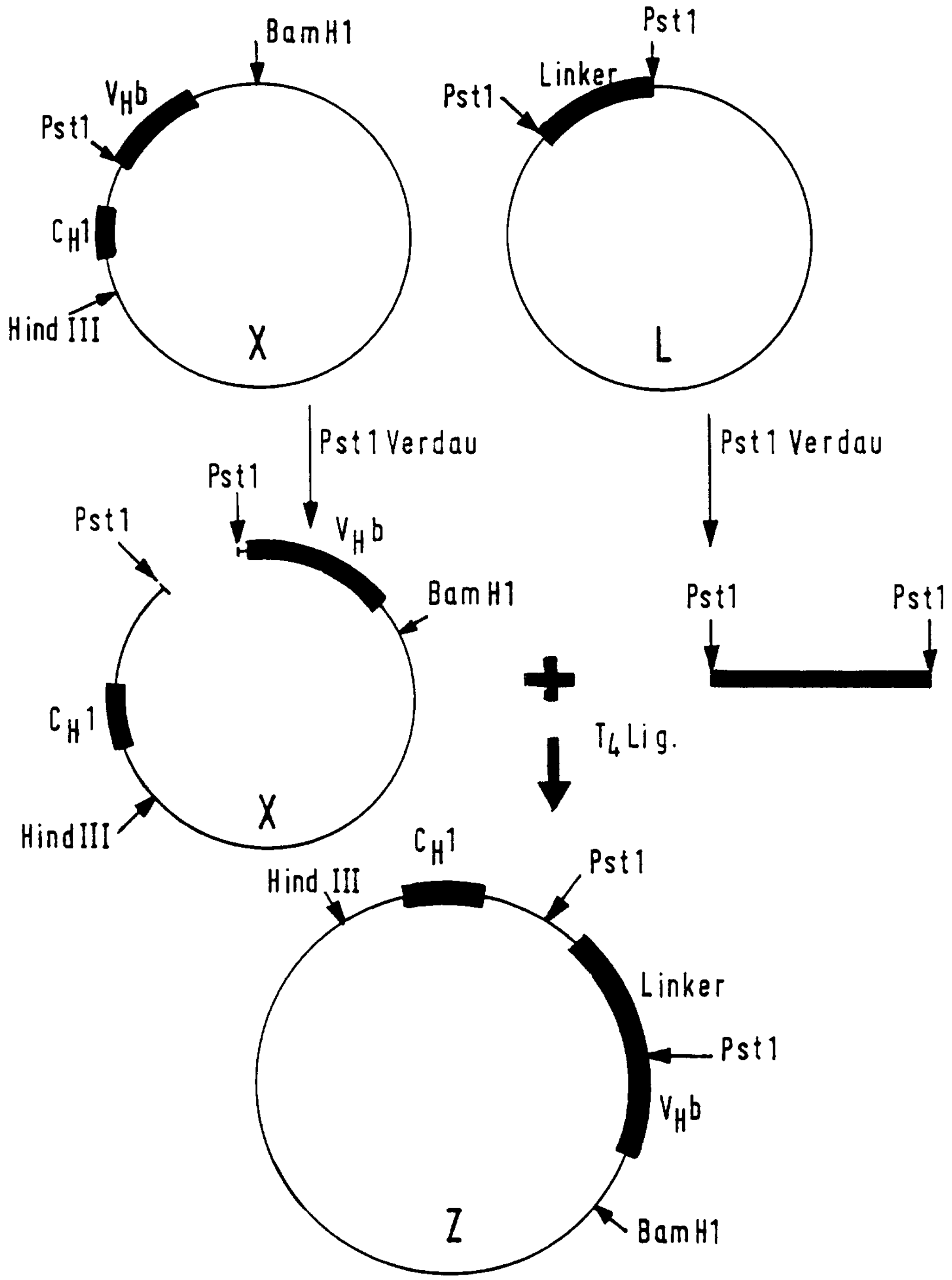
BamH1
$V_H$b
Pst1
$C_H$1
Hind III
X
Pst1
Linker
Pst1
L
Pst1 Verdau
Pst1
Pst1
$V_H$ b
BamH1
$C_H$1
HindIII
X
Pst1 Verdau
Pst1
Pst1
+
$T_4$ Lig.
$C_H$1
Hind III
Pst1
Linker
Pst1
$V_H$b
Z
BamH1

FIG. 12

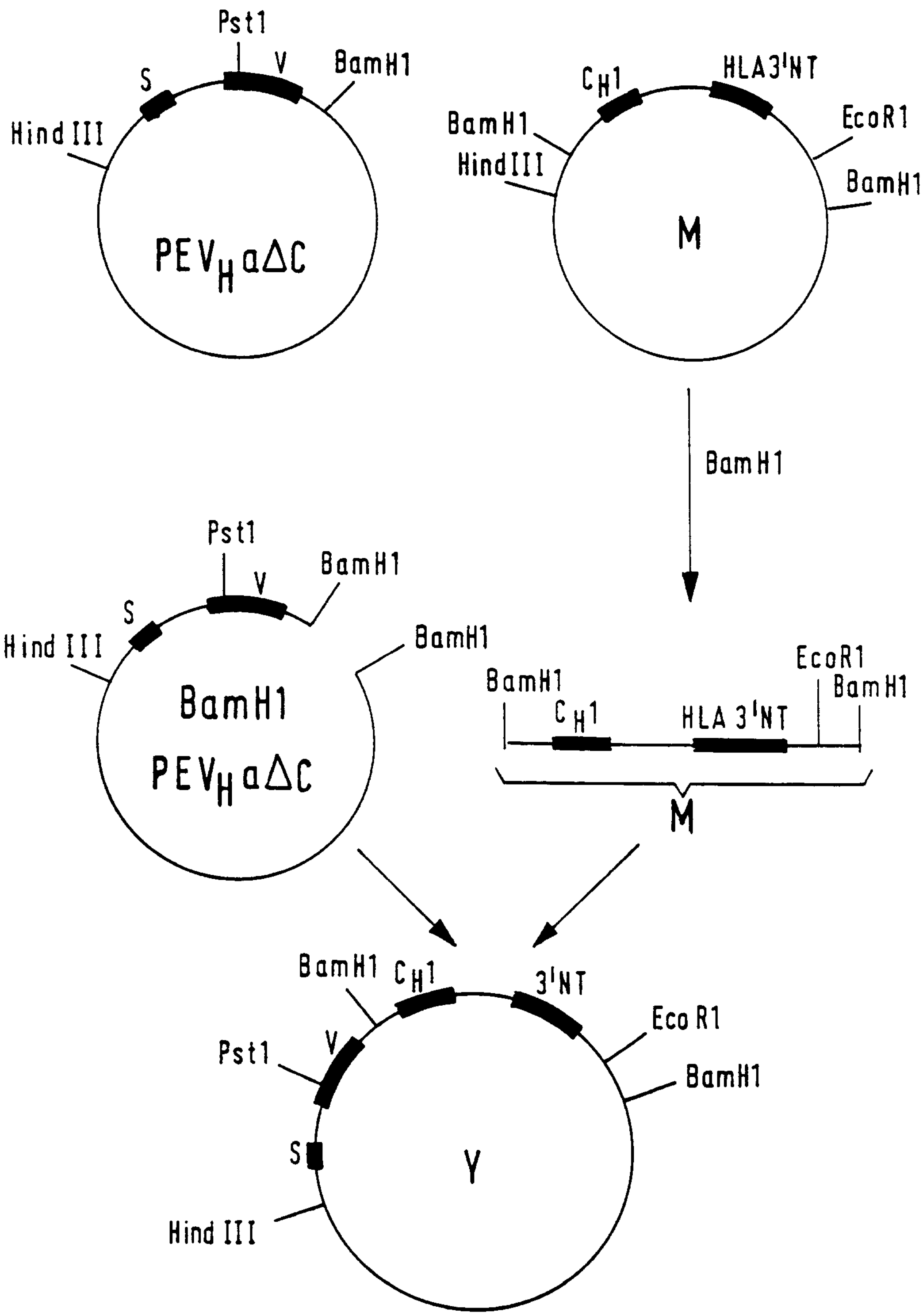
Pst1
V
BamH1
S
Hind III
PEV$_H$a$\Delta$C
C$_H$1
HLA3'NT
EcoR1
BamH1
Hind III
M
BamH1
Pst1
V
BamH1
S
Hind III
BamH1
BamH1
PEV$_H$a$\Delta$C
EcoR1
BamH1
BamH1
C$_H$1
HLA 3'NT
M
BamH1
C$_H$1
3'NT
Eco R1
Pst1
V
BamH1
S
Y
Hind III

FIG.13

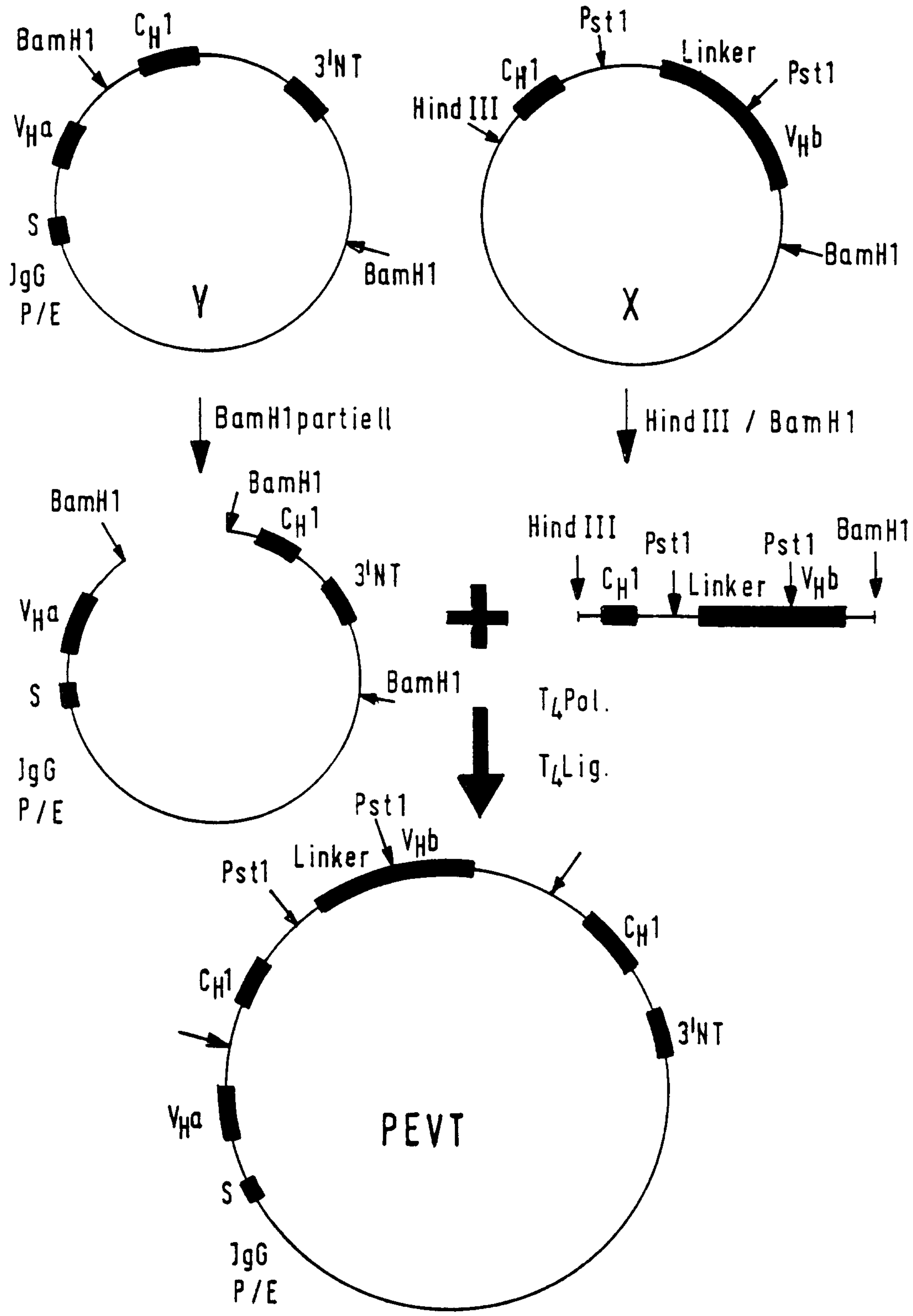
BamH1
$C_H1$
3'NT
$V_Ha$
S
JgG
P/E
BamH1
Y
Pst1
Linker
$C_H1$
Pst1
Hind III
$V_Hb$
BamH1
X
BamH1partiell
HindIII / BamH1
BamH1
BamH1
$C_H1$
3'NT
$V_Ha$
S
BamH1
JgG
P/E
Hind III
Pst1
Pst1
BamH1
$C_H1$
Linker
$V_Hb$
$T_4$Pol.
$T_4$Lig.
Pst1
Linker
$V_Hb$
Pst1
$C_H1$
$C_H1$
3'NT
$V_Ha$
PEVT
S
JgG
P/E

FIG.14

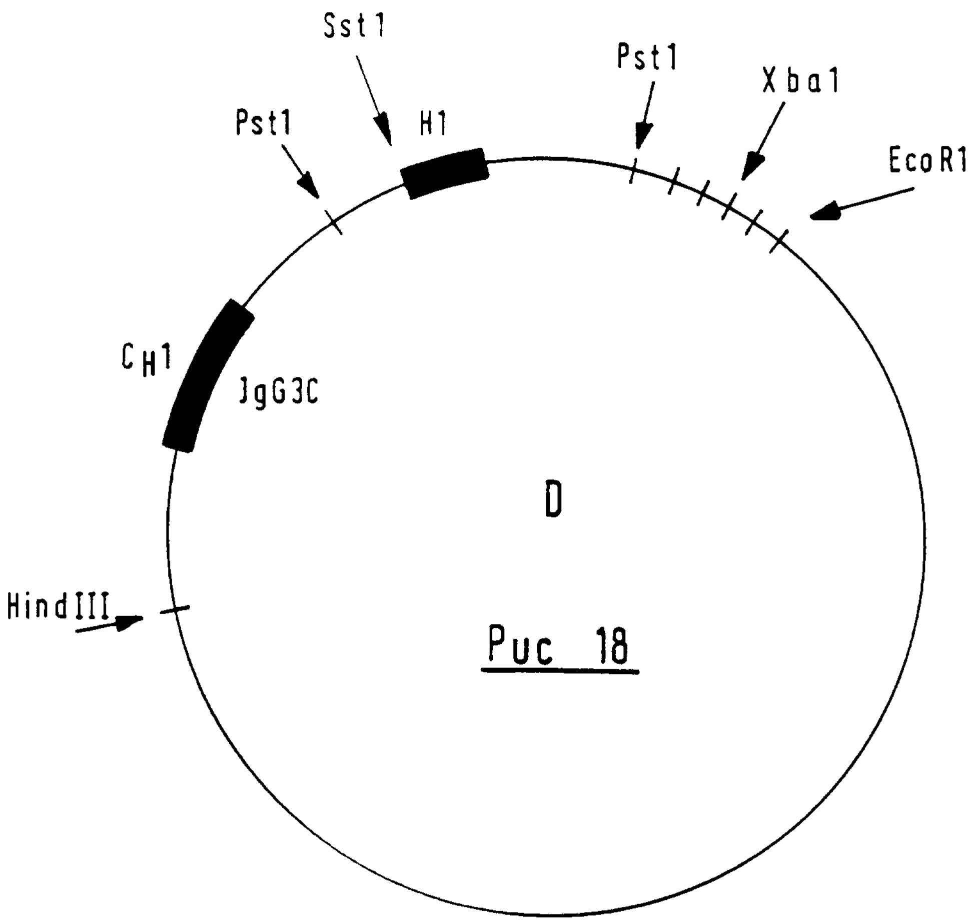

FIG. 15

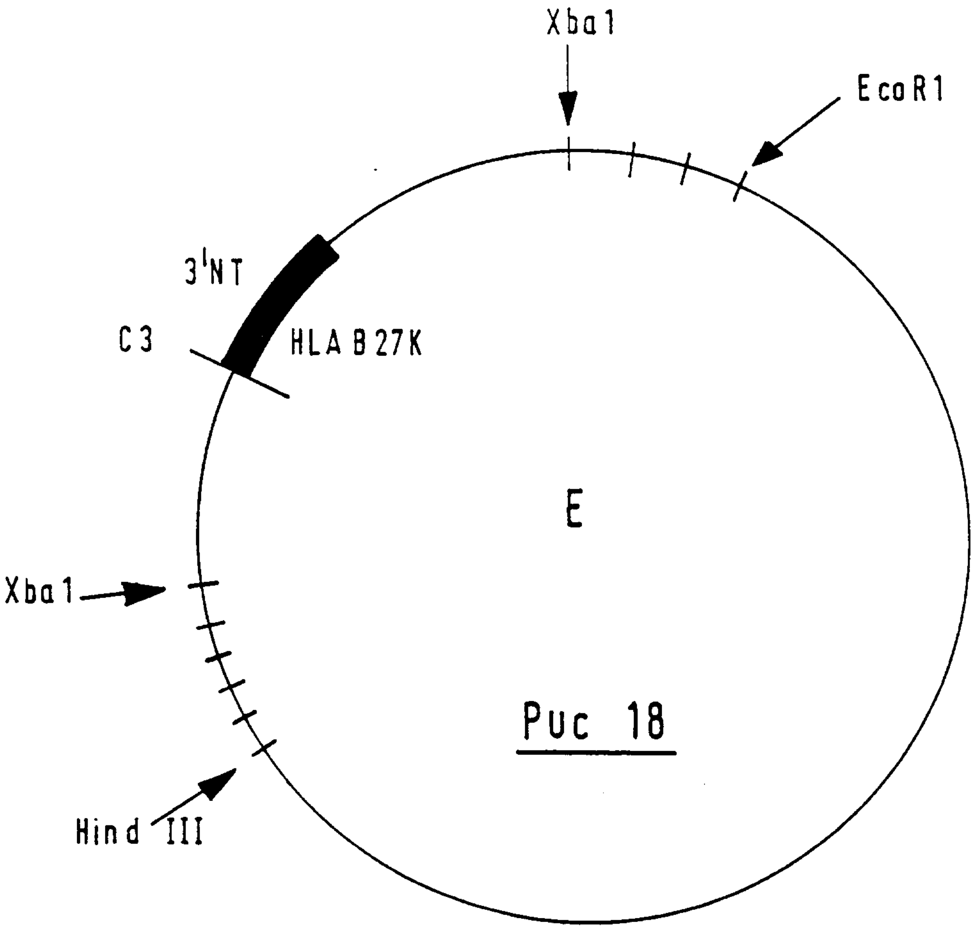

FIG.16

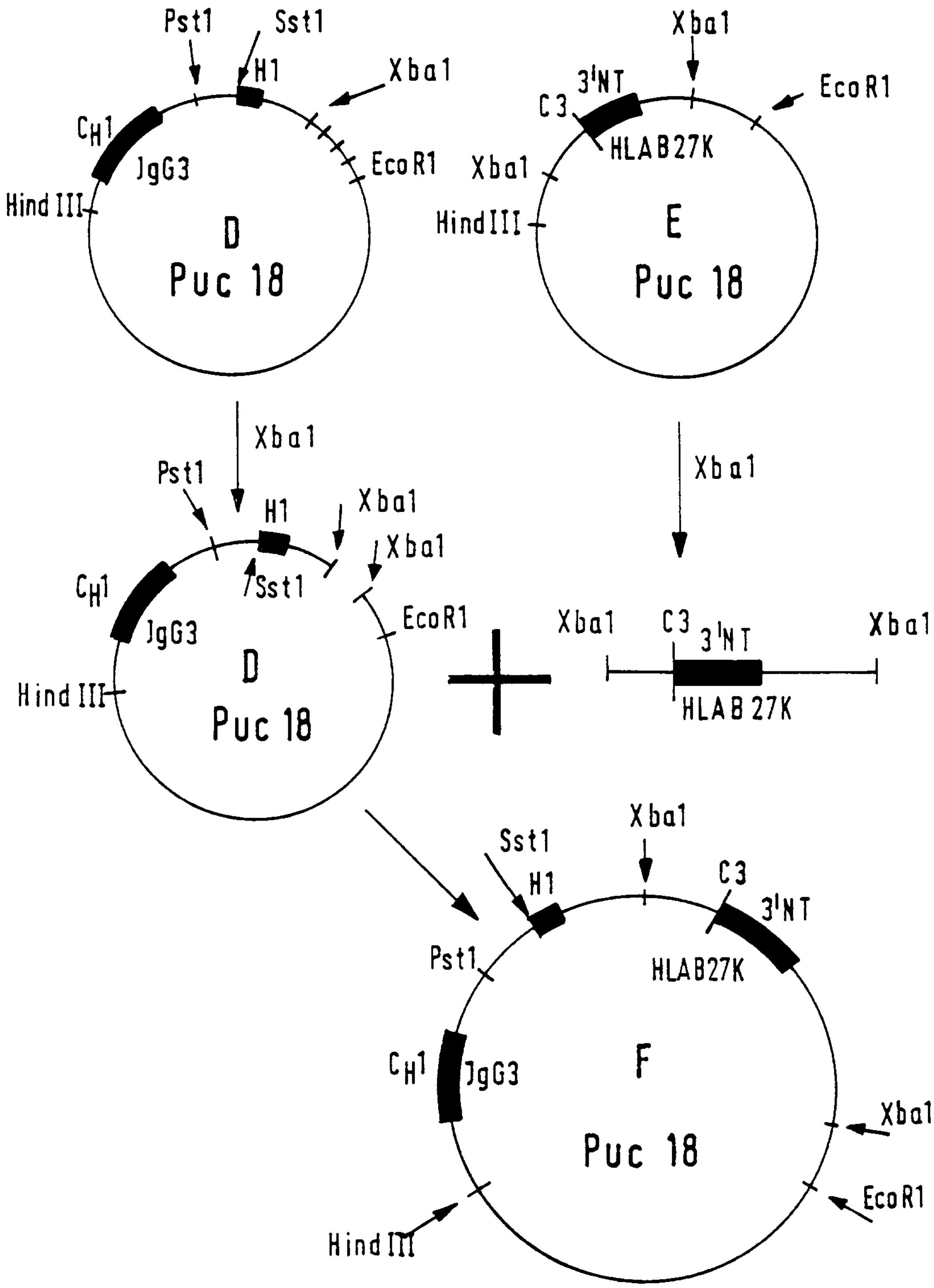
Pst1
Sst1
H1
Xba1
CH1
JgG3
EcoR1
Hind III
D
Puc 18
Xba1
3'NT
C3
HLAB27K
EcoR1
Xba1
Hind III
E
Puc 18
Xba1
Xba1
Pst1
H1
Xba1
Xba1
Sst1
CH1
JgG3
EcoR1
Hind III
D
Puc 18
Xba1
C3
3'NT
Xba1
HLAB 27K
Xba1
Sst1
H1
C3
3'NT
Pst1
HLAB27K
CH1
JgG3
F
Puc 18
Xba1
EcoR1
Hind III

FIG.17

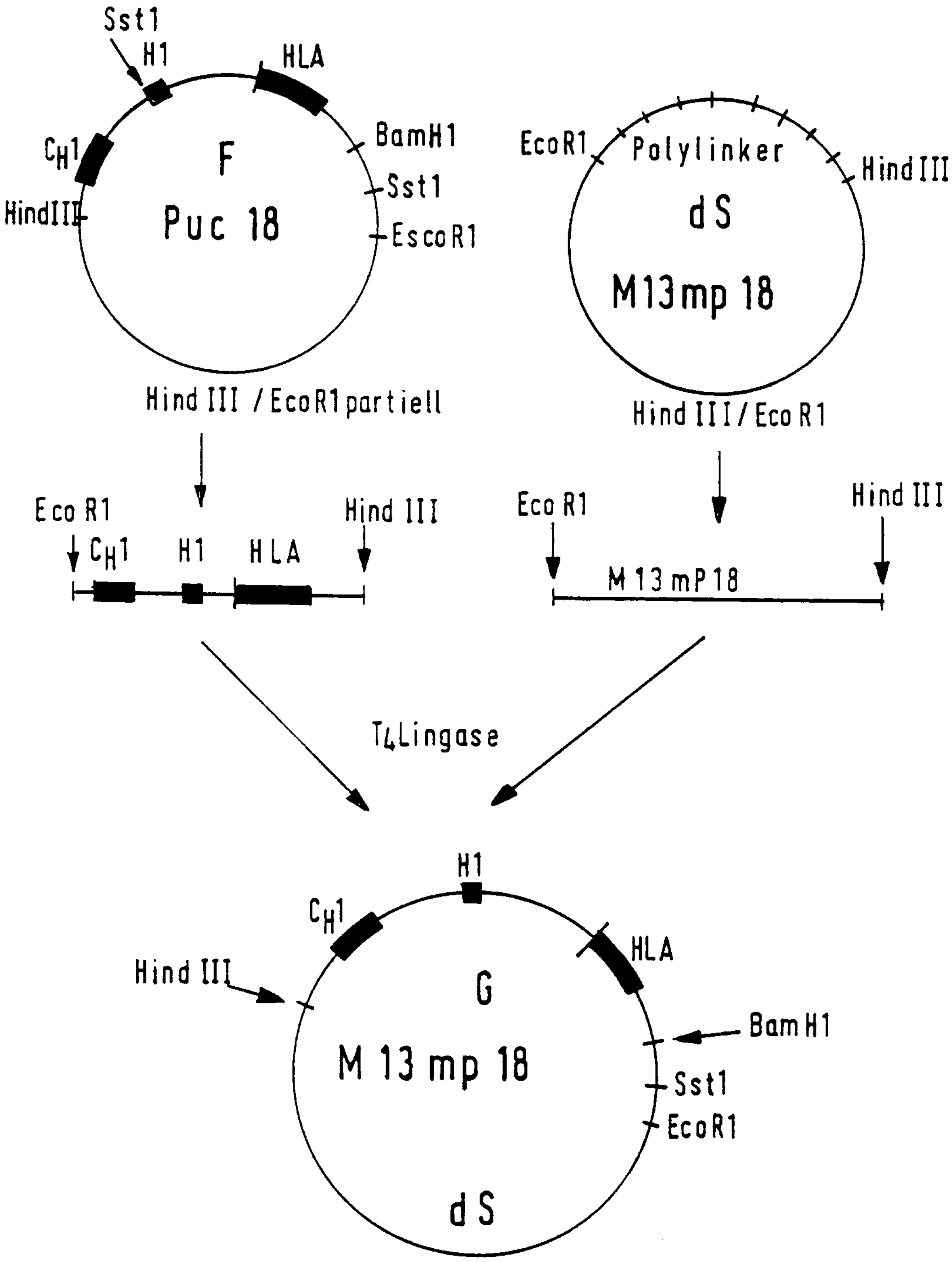
Sst1
H1
HLA
$C_H1$
F
BamH1
Sst1
HindIII
Puc 18
EscoR1
EcoR1
Polylinker
HindIII
dS
M13mp 18
Hind III / EcoR1 partiell
Hind III / Eco R1
Eco R1
$C_H1$
H1
HLA
Hind III
Eco R1
Hind III
M 13 mP 18
T4 Lingase
H1
$C_H1$
HLA
Hind III
G
Bam H1
M 13 mp 18
Sst1
EcoR1
dS

FIG. 18

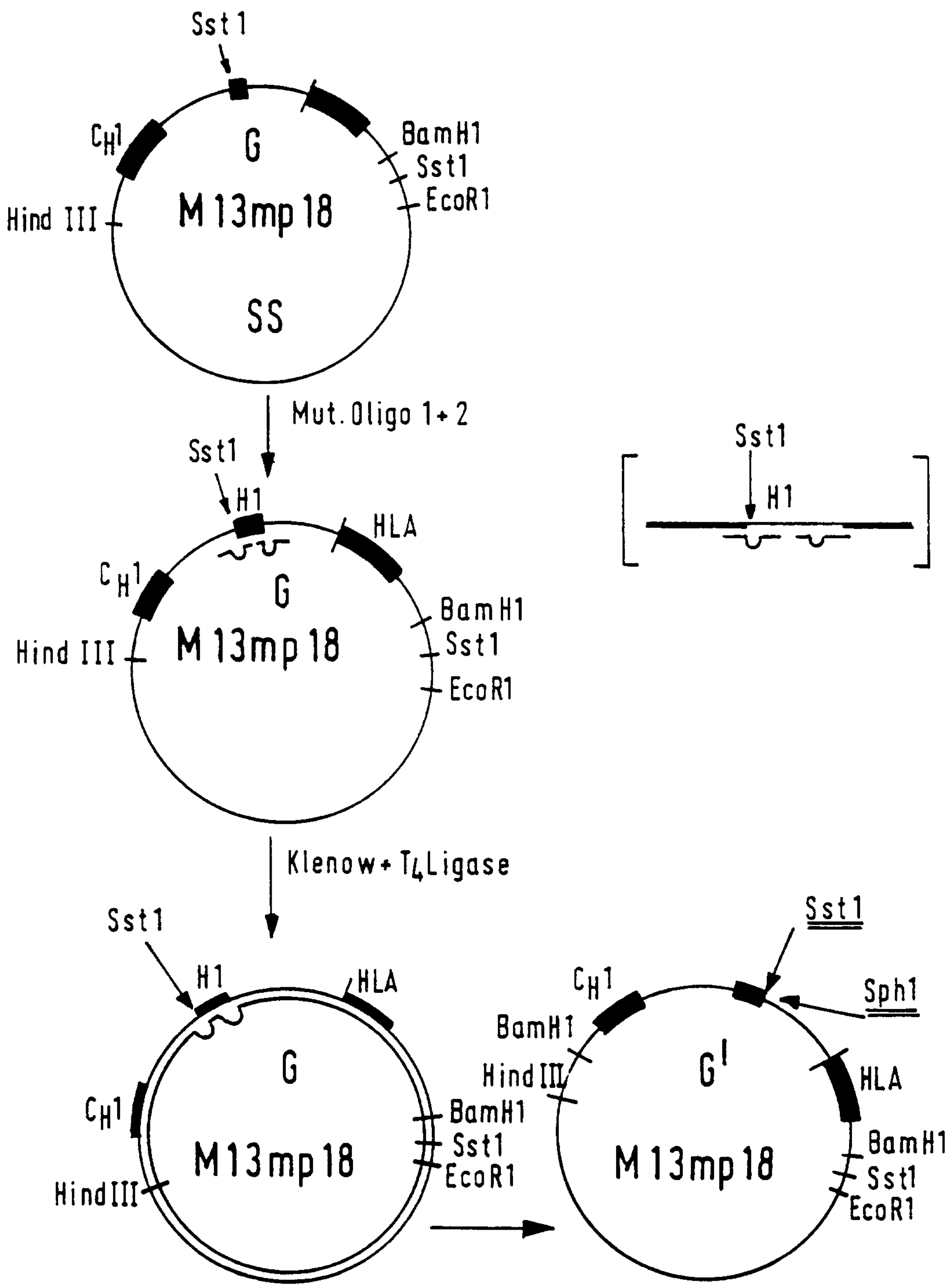
Sst1
G
$C_H1$
BamH1
Sst1
EcoR1
Hind III
M13mp18
SS
Mut. Oligo 1+2
Sst1
H1
HLA
Klenow + $T_4$Ligase
G'
Sph1

FIG. 19

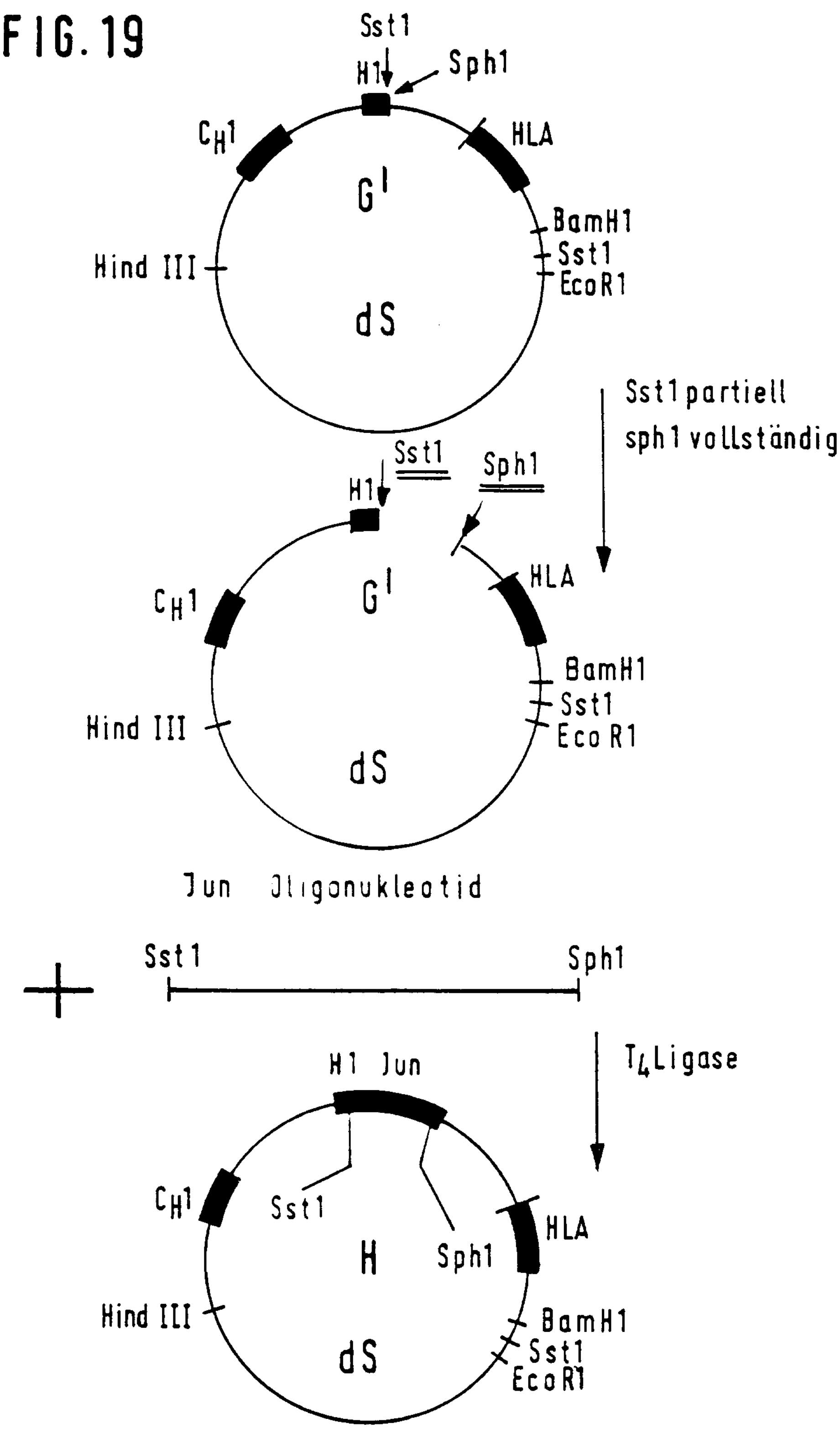
Sst1
H1
Sph1
$C_H1$
HLA
G'
BamH1
Sst1
EcoR1
Hind III
dS
Sst1 partiell
sph1 vollständig
Sst1
Sph1
H1
$C_H1$
HLA
G'
BamH1
Sst1
Eco R1
Hind III
dS
Jun Oligonukleotid
Sst1
Sph1
$T_4$ Ligase
H1 Jun
$C_H1$
Sst1
HLA
H
Sph1
Hind III
BamH1
Sst1
EcoR1
dS

FIG. 20

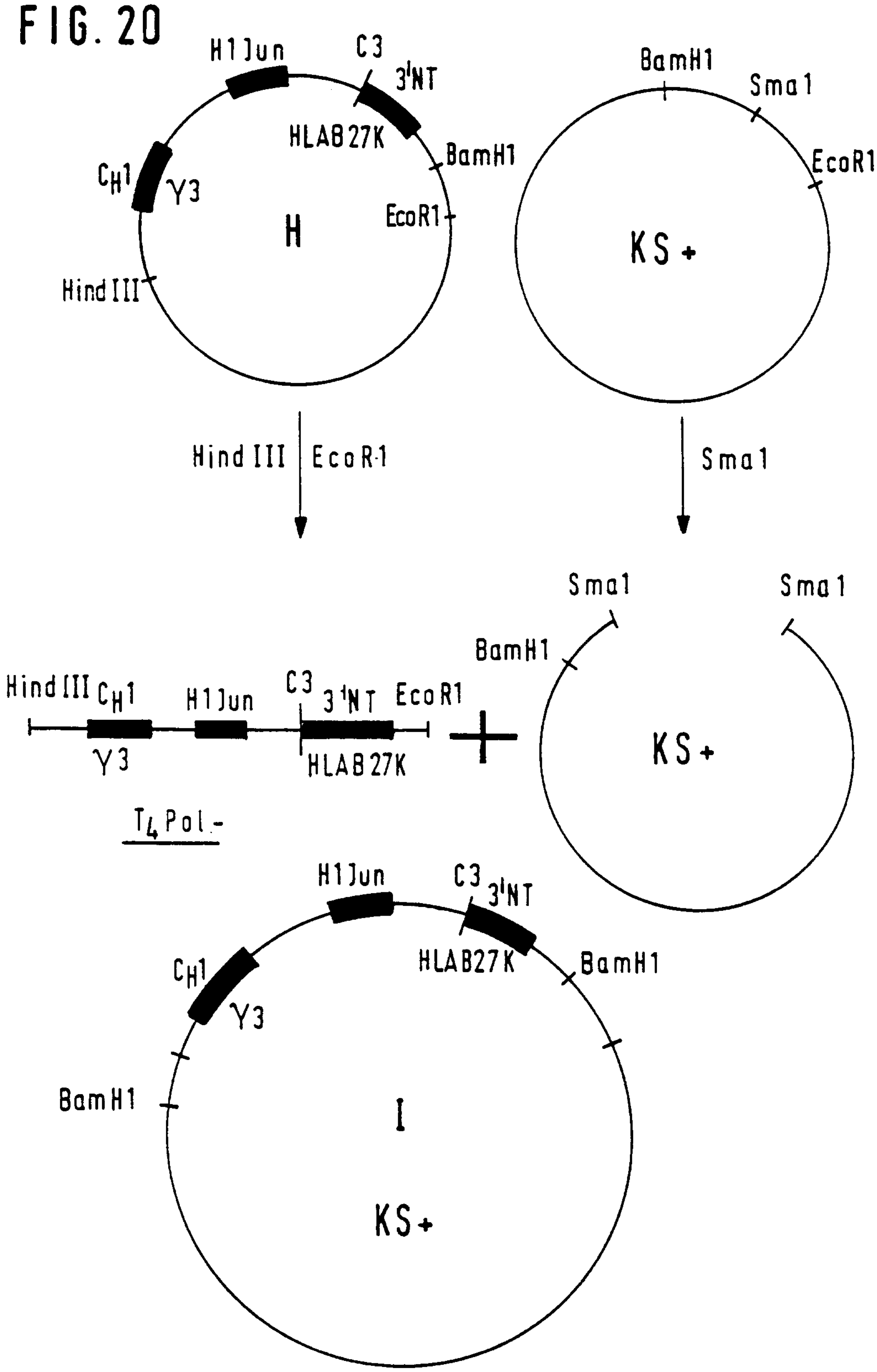

H1Jun
C3
3'NT
HLAB27K
BamH1
$C_H1$
γ3
H
EcoR1
HindIII
BamH1
Sma1
EcoR1
KS+
Hind III
EcoR1
Sma1
Sma1
Sma1
BamH1
HindIII $C_H1$
H1Jun
C3
3'NT
EcoR1
γ3
HLAB27K
KS+
$T_4$ Pol.-
H1Jun
C3
3'NT
$C_H1$
HLAB27K
BamH1
γ3
BamH1
I
KS+

FIG. 21

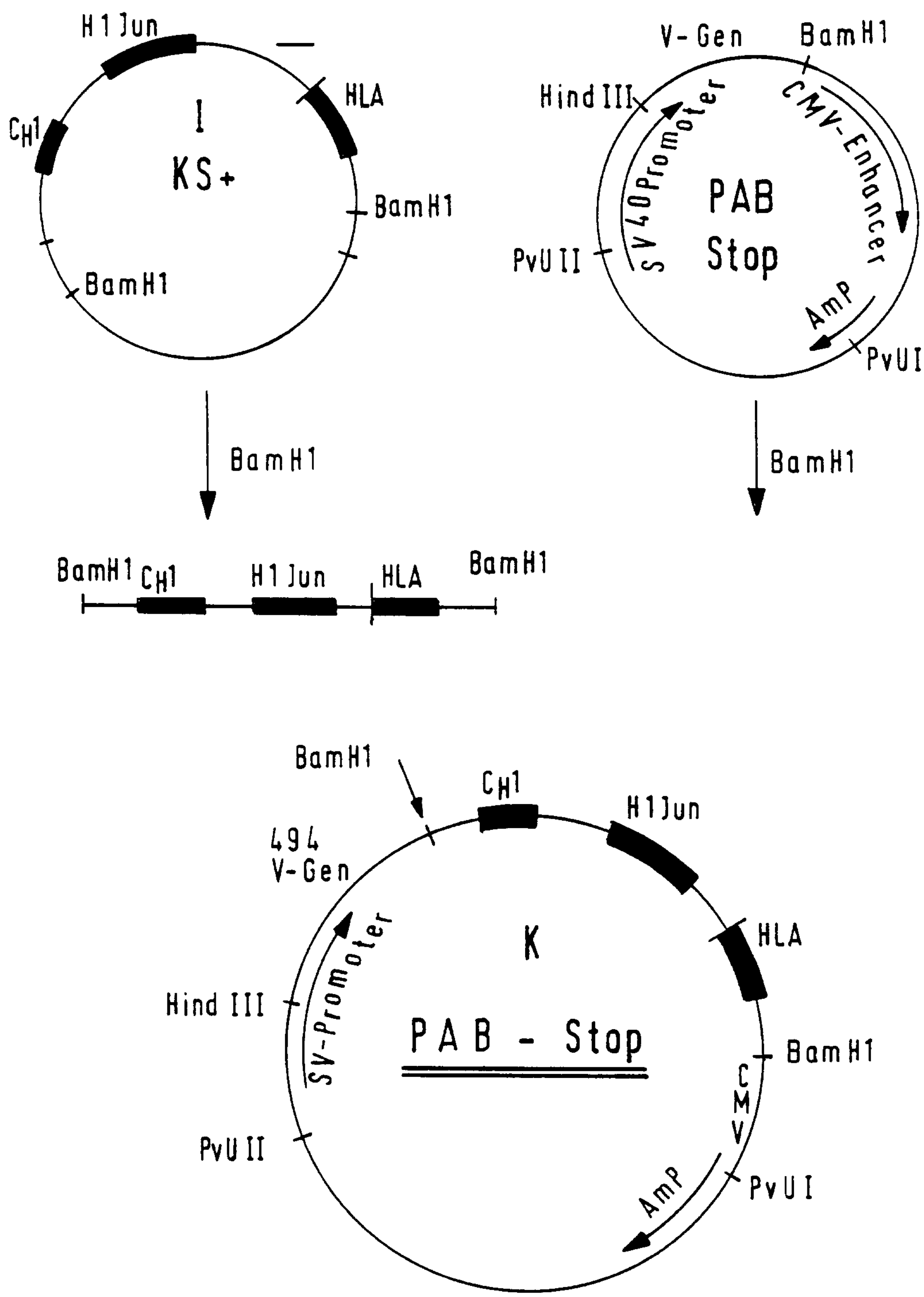
H1Jun
HLA
CH1
I
KS+
BamH1
BamH1
V-Gen
BamH1
Hind III
SV40Promoter
CMV-Enhancer
PAB
Stop
PvU II
AmP
PvUI
BamH1
BamH1
BamH1
CH1
H1Jun
HLA
BamH1
BamH1
CH1
H1Jun
494
V-Gen
SV-Promoter
K
HLA
Hind III
PAB - Stop
BamH1
CMV
PvU II
AmP
PvU I